# EUROPEAN PATENT APPLICATION

(11) **EP 4 714 272 A1**
(43) Date of publication of application: **25.03.2026**
(21) Application number: 24201131.0
(22) Date of filing: 18.09.2024
(51) Int. Cl.: A23L 33/20, A23L 33/00

(54) **DIET COMPOSITION FOR THE PREVENTION AND/OR THE TREATMENT OF HYPOSMIA AND HYPOGEUSIA**

(71) Applicant: L-Nutra Inc., Culver City, CA 90230 (US)
(72) Inventor: ALESSANDRINI, Marco, 00044 Frascati (RM) (IT); MICARELLI, Alessandro, 00065 Fiano Romano (RM) (IT); LONGO, Valter, Culver City, CA 90230 (US); SCHIRANO, Fabrizio, Culver City, CA 90230 (US)
(74) Representative: Botti & Ferrari S.p.A.

(57) **Abstract**

Diet composition for use in the prevention and/or the treatment of hyposmia and/or hypogeusia in a human subject, the diet composition comprising: a) a fasting mimicking diet component to be administered for a first time period, said fasting mimicking diet component providing less than 50% of the normal caloric intake of the subject with both protein restriction and sugar restriction; and b) a re-feeding diet component to be administered for a second time period, said re-feeding diet component providing 60 -100% of the normal caloric intake of the subject, wherein the fasting mimicking diet component and the re-feeding diet component are administered for multiple cycles.

## Description

### Technical Field

The present invention relates to the technical field of the pharmaceutical and dietary industries.

In particular, the invention refers to a diet composition for the prevention and/or the treatment of hyposmia and/or hypogeusia.

### Prior art

Obesity is one of the major risk factors for age-related diseases and has rapidly grown in the US, Europe and many countries. Efforts to reverse this epidemic have been largely unsuccessful, in part because of lifestyle changes that are not sustainable for the majority of patients.¹ Chemosensory perception - i.e. taste and smell - is a pivotal contributor of food palatability in humans, that plays a significant role in food choice and energy consumption.² In turn, nutrients intake or food habits may impact on taste and smell sensitivity,³ since the tongue and olfactory bulb are obesity-associated organs, and their function is affected by the biochemical regulators promoting obesity.⁴ As a result, changes in taste perception have been associated with an increase in body mass index (BMI) ^{3,5,6} and olfactory impairment has been found in overweight (OW) and obese subjects.^{7,8}

In particular, the hyposmia is one of the common disorders of obese subjects. Hyposmia, or microsmia, is a reduced ability to smell and to detect odors.

Because of the close link between taste and smell, many people suffering hyposmia also develop hypogeusia, a decreased sense of taste.

Indeed, while with hyposmia patients usually have a normal perception of salty, sweet, sour, and bitter substances, they cannot efficiently discriminate between flavors, since this greatly depends on normal olfaction.

Notably, many factors, for example high cholesterol, low insulin, low ghrelin and high leptin serum levels, are collectively associated with a dampening effect on olfactory and gustatory performance in obese individuals.^{4,5,7,8,14}

The technical problem underlying the present invention is therefore providing a remedy that may lead to treat and/or prevent hyposmia and hypogeusia to enhance or preserve the olfactory and gustatory performance of subjects, in particular subjects with an excess of weight.

### Summary of the Invention

This problem has been solved by providing a diet composition for use in the prevention and/or the treatment of hyposmia and/or hypogeusia in a human subject, the diet composition comprising:
a fasting mimicking diet component to be administered for a first time period, said fasting mimicking diet component providing less than 50% of the normal caloric intake of the subject with both protein restriction and sugar restriction; and
a re-feeding diet component to be administered for a second time period, said re-feeding diet component providing 60-100% of the normal caloric intake of the subject;
wherein the fasting mimicking diet component and the re-feeding diet component are administered for multiple cycles.

Preferably, the diet composition is administered to a human subject with Body Mass Index (BMI) ≥ 25.

Specifically, the diet composition is administered to a human subject with Body Mass Index (BMI) equal to or higher than 25 and lower than 40, preferably a BMI between 25 and 40, more preferably 30 and 40.

In particular, said human subject is between from 18 to 75 years old.

Preferably, said human subject does not suffer or has not suffered from any kind of chemosensory perception disturbances related to previous COVID-19 infection.

Preferably, the first time period is from 2 days to 10 days, preferably 2 to 6 days, more preferably the first time period being 5 days, and/or said second time period is from 7 to 85 days, preferably 25-26 days.

Within the meaning of the present invention, with the term "normal caloric intake of the subject" it is intended the number of calories that the subject consumes in order to maintain its weight.

The normal caloric intake of the subject can be estimated by interviewing the subject and/or by considering the subject weight.

As a rough guide, the normal caloric intake of the subject is on average 2600 kcal/day for men and 1850 kcal/day for women.

After the administration of the fasting mimicking diet component and optionally before it, said human subject observes a treatment with a re-feeding diet component, namely the patient follows its routinary eating.

Within the meaning of the present invention, with the term "routinary eating" it is intended that, in particular during said second time period, human subjects are not requested to follow any specific pre-determined diet regime.

For example, during said second time period human subjects are free to follow any balanced diet with a caloric intake proportional to their gender, age, height and physical activity routine, in order to maintain their weight.

Preferably, said re-feeding diet component provides 70-100%, more preferably 90-100%, of the normal caloric intake of the subject.

Typically, in the FMD protocol, the subjects' usual diet is replaced for a predefined number of days (e.g. 5 days), just once a month, during which they take the fasting-mimicking diet component and possibly drink plenty of water. In the the following 25-26 days they receive the refeeding diet component.

In one embodiment according to the present invention, the fasting mimicking diet component is provided for 5 days per month and the re-feeding diet component is provided for 25-26 days per month for six consecutive cycles.

Preferably, the fasting mimicking diet component and the re-feeding diet component are administered for multiple cycles for at least 6 months, preferably for 6-12 months.

Preferably, the blood concentration of leptin is reduced by 5 to 18 ng/ml and/or the blood concentration of ghrelin is increased by 30 to 90 pg/ml as measured after 6 months from the start of the administration of the diet composition according to the present invention for multiple cycles when compared to the blood concentration of leptin and/or ghrelin as measured before starting the administration of said diet composition, respectively.

Preferably, the blood concentration of leptin is between 8 and 20 ng/ml and/or the blood concentration of ghrelin is between 220 and 290 pg/ml as measured after 6 months from the start of the administration of the diet composition according to the present invention for multiple cycles, when compared to the blood concentration of leptin and/or ghrelin as measured before starting the administration of said diet composition, respectively.

Preferably, the fasting mimicking diet component provides the subject with no more than 1200 kcal/day, more preferably it provides the subject 600-1100 kcal/day.

Preferably, the first time period is 5 days and said fasting mimicking diet component provides the subject with 800-1200 kcal/day for the first day of said first time period and with 600-800 kcal/day for the second to the fifth day of said first time period.

Preferably, the fasting mimicking diet component provides the subject with no more than 11 kcal/kg of body weight/day, more preferably 2-8 kcal/kg of body weight/day.

In one embodiment, the fasting mimicking diet component provides the subject with a protein amount less than or equal to 36 g/day, preferably 0-20 g/day.

In particular, the fasting mimicking diet component provides the subject with a protein amount equal to 36, 20, 10, or 5 or 0 g/day, in increasing order of preference.

According to a preferred embodiment, if carbohydrates are present in the fasting mimicking diet component, they provide no more than half of the calories provided by the aforementioned diet component.

Preferably, the fasting mimicking diet component comprises proteins in an amount that is less than 15%, more preferably less than 12%, of the total calories provided by the fasting mimicking diet component.

Preferably, the fasting mimicking diet component comprises sugars in an amount that is less than 50%, more preferably less than 48%, of the total calories provided by the fasting mimicking diet component.

Preferably, said fasting mimicking diet component comprises fats in an amount that is equal to or more than 40%, more preferably less than 50%, even more preferably less than 48%, of the total calories provided by the fasting mimicking diet component.

Alternatively, said fasting mimicking diet component comprises fats in an amount that is between 40% - 80% of the total calories provided by the fasting mimicking diet component.

Preferably, the fasting mimicking diet component comprises at least 45% calories from fatty acids, up to 5% calories from proteins, in particular plant-based proteins, and up to 50% calories from carbohydrates.

In one embodiment, the fasting mimicking diet component provides the human subject with the following energy intake, as reported in following Table 1:

**Table 1**

| | Day 1 | Days 2, 3, 4, 5 |
|---|---|---|
| Total calories (kcal) | 800-1200 | 600-800 |
| Fats | 40-48% | 40-47% |
| Carbohydrates | 40-45% | 45-50% |
| Protein | 9-13% | 7-11% |

Preferably, said fasting mimicking diet component comprises complex carbohydrates from plant sources, which more preferably comprise soy, rice or other cereals.

Preferably, in said fast mimicking diet component at least 50% of the calories from fatty acids are from coconut oil and tree nuts. The latter more preferably comprise walnuts, macadamia nuts and/or almonds.

Preferably, said fast mimicking diet component comprises a high content of monounsaturated and polyunsaturated fats and, as mentioned above, a reduced content of proteins and sugars.

Examples of FMD can be found in WO 2014/066426 and WO 2014/12700 publications.

Avantageously, FMD cycles were shown to reduce glucose, insulin-like growth factor 1 (IGF-1) and insulin plasma levels, ameliorate lipid profile, decrease visceral fat and modulate pro-inflammatory cytokines, particularly in subjects with high baseline levels of these markers.

In particular, by virtue of said fasting mimicking diet component, the human subject is fed with foodstuffs with a high content of monounsaturated and polyunsaturated fats and a reduced content of proteins and sugars (≥ 40% calories coming from fat, as mentioned above). A diet based on these specific nutritional and caloric intake has beneficial effects that are similar to those of fasting.

Advantageously, as shown in detail in the experimental section of this disclosure, by virtue of the administration of the diet composition according to the present invention, taste and smell sensitivity improve and this improvement is also detectable even several months after the end of the aforementioned multiple cycles.

Additionally, said fast mimicking diet component allows to reduce leptin and increase ghrelin.

Interestingly, ghrelin, leptin and insulin continued to be significantly different from baseline in FMDtoControl after the follow up 6-months period, leading to the hypotheses that either patients have altered their nutritional habits after the FMD intervention and/or that many of the effects of the FMD are long lasting.

As known, leptin and ghrelin levels have an influence on the regulation of obesity and, consequently, on the chemosensory regulation, in particular olfactory and gustatory regulation. In fact, the reduction of leptin and the increase of ghrelin allows to reduce appetite and increase fullness, therefore helping in the reduction of body weight.

It is known that the imbalance of leptin and ghrelin affects obesity.⁹ Obese individuals generally exhibit a higher circulating concentration of leptin than normal weight individuals due to their higher percentage body fat.

Accordingly, it is also disclosed a diet composition for use in the prevention and/or the treatment of hyposmia and/or hypogeusia in a human subject, the diet composition comprising a fasting mimicking diet component to be administered for a preset time period.

Preferably, the diet composition is administered to a human subject with Body Mass Index (BMI) ≥ 25.

Specifically, the diet composition is administered to a human subject with Body Mass Index (BMI) equal to or higher than 25 and lower than 40, preferably a BMI between 25 and 40, more preferably 30 and 40.

Specifically, said fasting mimicking diet component provides less than 50% of the normal caloric intake of the subject with both protein restriction and sugar restriction, wherein the fasting mimicking diet component is administered for multiple cycles.

Preferably, the preset time period is from 2 days to 10 days, preferably 2 to 6 days, more preferably the preset time period being 5 days, the multiple cycles comprising one administration once a month for at least 6 months, preferably for 6-12 months.

In one embodiment according to the present invention, the fasting mimicking diet component is administered for a predefined number of days (e.g., 5 days), just once a month. The fasting mimicking diet component is not provided for the subsequent 25-26 days.

Preferably, the fasting mimicking diet component is administered for multiple cycles for at least 6 months, more preferably for 6-12 months.

Preferably, the fasting mimicking diet component provides the subject with no more than 1200 kcal/day, more preferably it provides the subject 600-1100 kcal/day.

Preferably, the preset time period is 5 days and said fasting mimicking diet component provides the subject with 800-1200 kcal/day for the first day of said preset time period and with 600-800 kcal/day for the second to the fifth day of said preset time period.

Preferably, the fasting mimicking diet component comprises at least 45% calories from fatty acids and up to 5% calories from proteins, in particular plant-based proteins, and a maximum of 50% calories from carbohydrates.

Preferably, the fasting mimicking diet component provides the human subject with the following energy intake, as reported in above Table 1.

According to the present invention, the aformentioned problem has been also solved by providing a diet composition for use in the prevention and/or the treatment of a disease or disorder or condition involving a leptin and/or ghrelin imbalance in a human subject, the diet composition comprising:
a fasting mimicking diet component to be administered for a first time period, said fasting mimicking diet component providing less than 50% of the normal caloric intake of the subject with both protein restriction and sugar restriction; and
a re-feeding diet component to be administered for a second time period, said re-feeding diet component providing 60-100% of the normal caloric intake of the subject;
wherein the fasting mimicking diet component and the re-feeding diet component are administered for multiple cycles.

Preferably, said disease or disorder or condition involving a leptin and/or ghrelin imbalance in a human subject is selected from overweight or obesity.

Preferably, the first time period is from 2 days to 10 days, preferably 2 to 6 days, more preferably the first time period being 5 days and/or said second time period is from 7 to 85 days, more preferably 25-26 days.

In one embodiment according to the present invention, the fasting mimicking diet component is provided for 5 days per month and the re-feeding diet component is provided for 25-26 days per month for six consecutive cycles.

Preferably, the fasting mimicking diet component and the re-feeding diet component are administered for multiple cycles for at least 6 months, preferably for 6-12 months.

Preferably, the blood concentration of leptin is reduced by 5 to 18 ng/ml and/or the blood concentration of ghrelin is increased by 30 to 90 pg/ml as measured after 6 months from the start of the administration ofthe diet composition according to the present invention for multiple cycles when compared to the blood concentration of leptin and/or ghrelin as measured before starting the administration of said diet composition, respectively.

Preferably, the blood concentration of leptin is between 8 and 20 ng/ml and/or the blood concentration of ghrelin is between 220 and 290 pg/ml as measured after 6 months from the start of the administration of the diet composition according to the present invention for multiple cycles, when compared to the blood concentration of leptin and/or ghrelin as measured before starting the administration of said diet composition, respectively.

Preferably, the fasting mimicking diet component provides the subject with no more than 1200 kcal/day, more preferably it provides the subject 600-1100 kcal/day.

Preferably, the first time period is 5 days and said fasting mimicking diet component provides the subject with 800-1200 kcal/day for the first day of said first time period and with 600-800 kcal/day for the second to the fifth day of said first time period.

Preferably, the fasting mimicking diet component comprises at least 45% calories from fatty acids and up to 5% calories from proteins, in particular plant-based proteins, and a maximum of 50% calories from carbohydrates.

Preferably, the fasting mimicking diet component provides the human subject with the following energy intake, as reported in above Table 1.

The present invention will be further described with reference to the enclosed drawings and to some embodiments, which are provided below for illustrative and non-limiting purposes.

### Brief description of the drawings

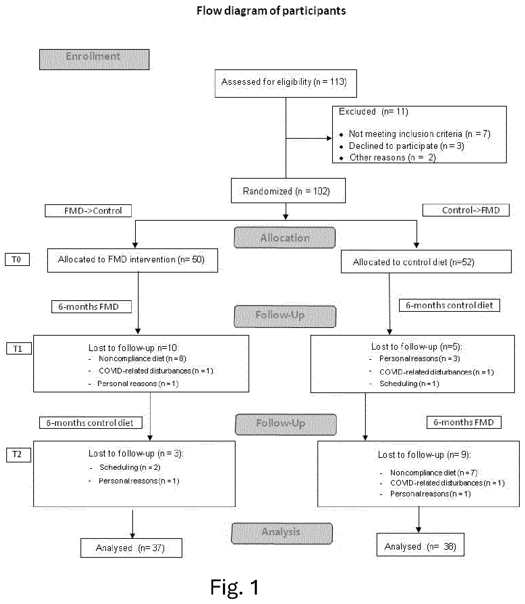

Figure 1 represents the Consolidated Standards of Reporting Trials (CONSORT) diagram of 113 subjects assessed for eligibility of which 102 were enrolled and randomized into two arms of the clinical study. Participants of the first group, named FMD4Control, (n = 50) started the fasting mimicking diet (FMD) after randomization. The FMD component is provided for 5 days per month for six consecutive cycles. The second group, named Control->FMD (n = 52), maintained their normal routinary eating for a 6-month monitoring period. After the first 6-month period participants were crossed-over to the FMD cycles regimen. Data were collected at enrollment (T0), after the first (T1) and second (T2) 6-month period. At the end of each 6-month FMD period data were collected on 5 days after subjects resumed their normal diet after the sixth FMD cycle.

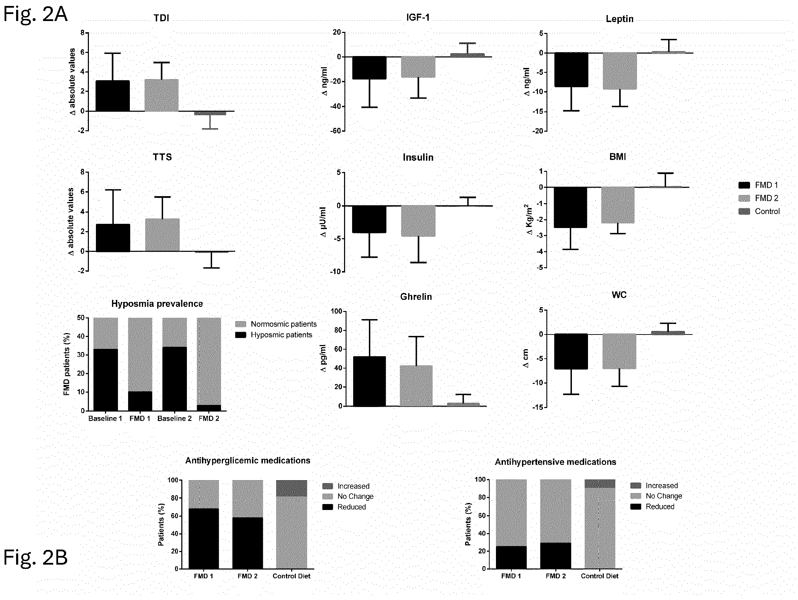

Figure 2 represents the chemosensory test of the two arms. In Figure 2A, the chemosensory testing and the biochemical assays differences (Δ) between the participants before and after FMD administration (n = 78, FMD 1 = 40 participants in FMD4Control, hence at T1, and FMD 2 = 38 participants in Control→FMD, hence at T2) and the partecipants when they observed the routinary eating (control) before and after the control 6-month period (n = 47 partecipants in Control->FMD at T1) are reported. In Figure 2B the percentage of participants who respectively changed antidiabetic and antihypertensive administration after 6 monthly fasting mimicking diets (FMD) cycles or routinary eating (control) are reported. All data are means ± SD. TDI, olfactory composite score; TTS, total taste score; ng, nanogram; ml, milliliter; %, percentage; FMD, fasting mimicking diet; T0, baseline; T1, after 6 monthly FMD.

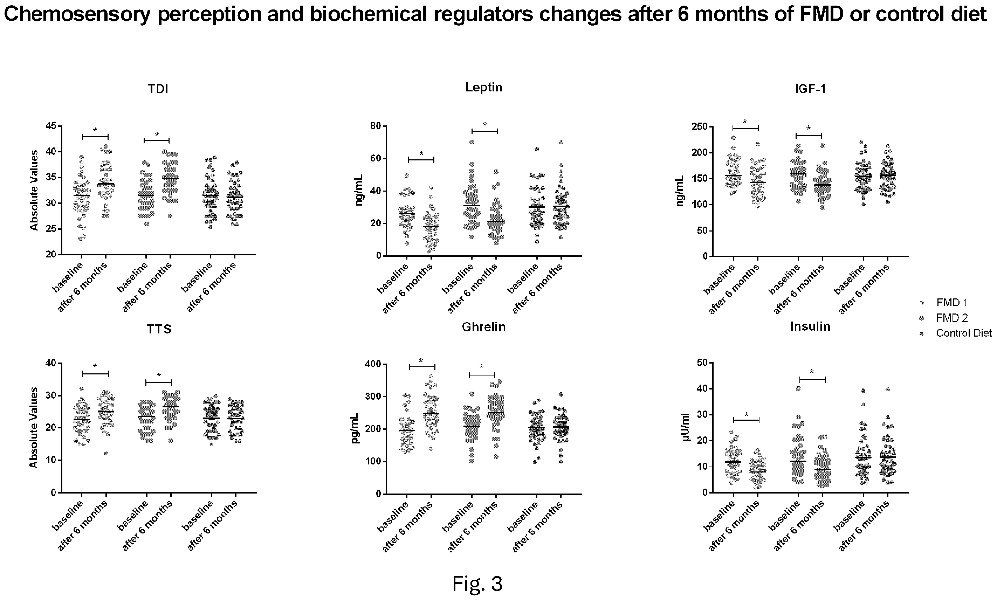

Figure 3 represents the scatter dot plot (line at median) depicting changes at baseline and after 6-month period in main chemosensory perception testing and biochemical regulators of control diet (n = 47), fasting mimicking diet (FMD) in FMD4Control (FMD 1, n = 40) and Control->FMD (FMD 2, n = 38) participants. Asterisks indicate significant within-subject differences (exact p-values are given in the text). ng, nanogram; pg, picogram; ml, milliliter; µU, micro international unit; TTS, total taste score; TDI, composite olfactory score. Exact p-values are given in the detailed description.

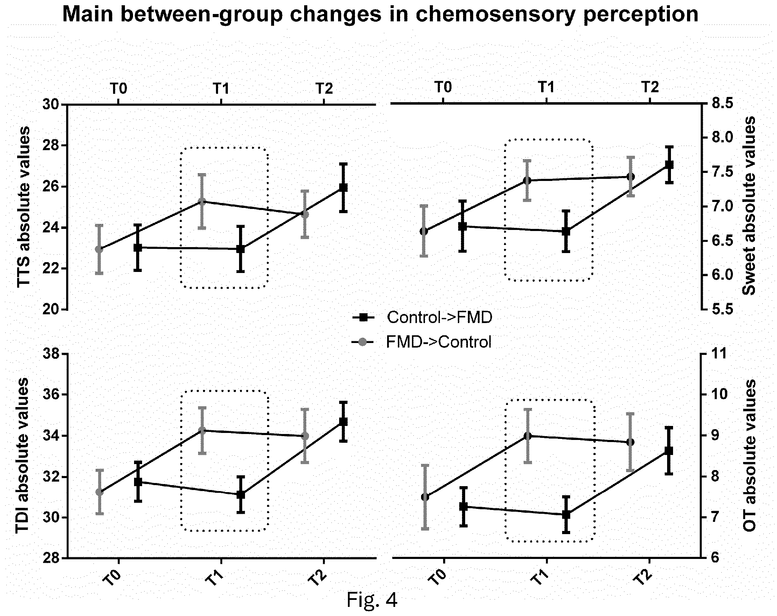

Figure 4 represents the main between-group changes in chemosensory perception testing at baseline (T0), after the first semester (T1) in which FMD4Control participants followed the fasting mimicking diet administration and Control→FMD participants maintained their regular eating and after the second semester (T2) in which FMD4Control and Control→FMD were crossed over. Dotted boxes indicate significant between-group differences (exact p-values are given in the detailed description). Values are given in mean with 95% CI. OT, odor threshold; TTS, total taste score; TDI, composite olfactory score.

### Detailed Description

The invention relates to a diet composition as described above for the prevention and/or the treatment of hyposmia. The diet composition is administered as described above to subject with BMI ≥ 25 and with age between 18 to 75.

For the purposes of this invention, the following acronyms have the following meanings:

### Acronyms

| | |
|---|---|
| Fast Mimicking Diet | FMD |
| Consolidated Standards of Reporting Trials | CONSORT |
| Time 0, began of the study | T0 |
| Time 1, after 6 months | T1 |
| Time 2, end of the study | T2 |
| Body Mass Index | BMI |
| Ear-nose-throat | ENT |
| TDI composite score | TDI |
| TTS composite score | TTS |
| Insulin-like growth factor | IGF-1 |
| Insulin-like growth factor binding protein | IGFBP-1 |
| Odor threshold | OT |
| Odor discrimination | OD |
| Odor identification | OI |
| Alanine aminotransferase | ALT |
| Aspartate aminotransferase | AST |
| Waist circumference | WC |
| Fat mass | FM |
| Skeletal muscle mass | MM |
| Visceral fat | VF |
| Homeostasis Model Assessment | HOMA |
| Erythrocyte Sedimentation Rate | ESR |

As previous reported, after the treatment with the fasting mimicking diet component, the subjects observe said re-feeding diet, namely the patients follow their routinary eating. In particular, during said second time period the patients are not requested to follow any specific pre-determined diet regime.

### Materials and Methods

### Eligible patients

113 Caucasian adults with BMI ≥ 25 were recruited.

Inclusion criteria were BMI ≥ 25 and age is between 18 - 75 years old. All the participants were screened with general clinical and ear-nose-throat (ENT) examination. Chemosensory perception disturbances related to previous COVID-19 infection were considered as pre-enrollment exclusion criteria. Current or recent smokers (< 3 years of abstinence) and individuals affected by allergies and history of ENT surgery (namely Otorhinolaryngology) were excluded. Legally incapacitated people were excluded; individuals suffering from major systemic or organ failure disorders including neurodegenerative, psychiatric and cardiovascular disorders, nondiabetic liver disease, diabetes mellitus type 1, pancreatogenic diabetes, or steroid-induced diabetes, as evaluated by medical history, physical and neuropsychological examination and routine blood tests were further excluded.

Other exclusion criteria were:
- acute infection/fever, history of cancer disease in the last 5 years prior to study,
- infectious hepatitis B, C, or E, HIV infection,
- autoimmune diseases or immunosuppressive therapy,
- participation in other interventional studies
- anemia or hematological disease,
- polyneuropathy (autoimmune, alcohol-induced, or vitamin B12 deficiency, collagenosis),
- pacemaker and food allergy (nuts, tomato, soja, or other ingredients enlisted in the diet program),
- gastrointestinal/eating disturbances and surgery (also detection of Helicobacter pylori excluded by a C13 urea breath test, but not history of appendectomy) and
- history of gustatory and/or smelling disorders,
- pregnant and currently breastfeeding females,^{4,7}
- participants suffering from anosmia (i.e. TDI ≤ 16.5),¹⁷
- participants demonstrating ageusia were excluded.

Furthermore, a vegetarian/vegan diet, ongoing use of medication with an impact on chemosensory perception and drugs/alcohol abuse were considered as exclusion criteria.

### Randomisation and masking

Eligible participants were randomly assigned to two different experimental group. In particular, one group began with Fasting Mimicking Diet (FMD) component, followed by the re-feeding component, and after 6 months (hence 6 cycles of administration of the diet component according to the invention) the subjects observed their routinary eating for other 6 consecutive months (FMD-Control). Another group began with their routinary eating and subsequently the subjects were treated with FMD component, followed by the re-feeding component, for 6 cycles of administration of the diet component according to the invention (Control-FMD).

One cycle of said treatment includes the administration of FMD component to the patient for 5 consecutive days and of a re-feeding component for the subsequent 25 days.

### Diet treatment

FMD component is a plant-based diet developed to obtain fasting-like reduction in serum glucose and Insulin-like growth factor (IGF-1), and an increase in Insulin-Like Growth Factor Binding Protein-1 (IGFBP-1) and ketone bodies while providing both macro- and micronutrients to minimize the burden of fasting and adverse effects.¹²

As mentioned, the FMD component was administered for 5 consecutive days (Prolon^{®} of L-Nutra, www.prolonfmd.com) and comprises formulations of vegetable-based food, namely soups, energy bars, energy drinks, chip snacks, tea, and a supplement providing high levels of minerals, vitamins, and essential fatty acids.

The fasting mimicking diet component on Day 1 provides the subjects with ∼4600 kJ, i.e 1099 kcal (11% protein, 46% fat, and 43% carbohydrate), whereas on days 2 to 5 provides the subjects with ∼3000 kJ, i.e. 717 kcal (9% protein, 44% fat, and 47% carbohydrate) per day.

All food items to be consumed per day were individually packed to allow the subjects to choose when to eat them while avoiding accidentally consuming components intended for the following day and reducing the risk of other sources of intake rather than the packed ones. The administration of the FMD component started on the first possible day after the baseline visit.

Oral antidiabetic therapy was discontinued during the administration of the FMD component.¹³ Antihypertensive medication was reduced in case of hypotension (systolic blood pressure lower than 100 mmHg and diastolic blood pressure lower than 60 mmHg). All participants were instructed to avoid excessive physical activity during the administration of the FMD component and to return to their normal physical activity afterward.¹³

The participants had to document any additional food or beverage items consumed during the five-day period beyond the provided meal kit.^{13,16}

### Primary outcomes

### Chemosensory Testing

Smell function was assessed by Olfactory function testing, namely the commercially available Sniffin' Sticks^{®} test battery (Sniffin' Sticks; Burghart Instruments, Wedel, Germany) in order to evaluate olfactory performance in clinical and research context.¹⁸ It includes subtests for odor threshold (OT), odor discrimination (OD) and odor identification (OI), which are associated with different aspects of olfactory processing along the neural stream from olfactory bulb to the olfactory cortex¹⁹. There was an interval of 3 to 5 minutes between consecutive subtests.²⁰ The score ranges from 0 to 16. The average of the three subtests results in the TDI compositescore and reflects the general olfactory capacity, which thus can range from 0 to 48, wherein the higher scores correspond to a greater funcionality.¹⁷ Furthermore, olfactory performances were categorized as abnormal when the TDI value is < 25th percentile based on normative data, wherein the values are age- and gender- based (cut-off values for females were 32.35, 33.5, 33.5, 32.5, 30.75, 29.13 and 25.5 and for male were 30.75, 32.75, 32.76, 30.44, 29.25, 28.5, 22.75, respectively for 11-20, 21-30, 31-40, 41-50, 51-60, 61-70 and 71-80 years sub-groups).^{21,22}

Furthermore, the groups participated to a taste test, namely Taste Strips. The taste test is a semi-quantitative, accurate, quick and easy tool to investigate the threshold of both sides of the tongue for each of the four basic tastants. The tastants are administered to patients at increasing concentrations (sweet: 0.05, 0.1, 0.2, 0.4 g/ml sucrose; sour: 0.05, 0.09, 0.165, 0.3 g/ml citric acid; salty: 0.016, 0.04, 0.1, 0.25 g/ml sodium chloride; bitter: 0.0004, 0.0009, 0.0024, 0.006 g/ml quinine hydrochloride). The taste test is a filter paper strip ("Taste Strips", Burghart Instruments, Wedel, Germany) impregnated with a specific amount of the four basic tastants, namely sweet, sour, salty and bitter taste²³. The tongues of the patients were extended, and the strips were applied on the left or on the right side of the anterior third of the tongue (32 trials). Before each test, the mouth was rinsed with water. The participants had to identify the taste from a list of the four tastants by a multiple forced choice method (i.e. the participant may indicate only one of the tastant reported in the list. In case of correct or uncorrect response a score equal to 1 or 0 is respectively assigned). The results were derived from the sum of the number of correctly identified tastants per side (ranging from 0 to 16 per side) and, subsequently, the left and right side scores were added in order to obtain the total number of identified tastant (TTS composite score) that can range from 0 to 32.²³ Taste performance were categorized as abnormal when the TTS value is < 10th percentile based on normative data, wherein the values are age- and gender- based (cut-off values for female were 19, 15 and 10.2 and for male were 17, 9 and 9 respectively for 18-40, 41-60 and > 60 years sub-groups).¹⁵

### Secondary outcomes

### Biochemical assays

Baseline laboratory parameters, including serum glucose, alanine aminotransferase (ALT) and aspartate aminotransferase (AST), total cholesterol, triglycerides (TGs), high density lipoprotein (HDL) cholesterol and low-density lipoprotein (LDL) cholesterol, C-reactive protein (CRP), erythrocyte sedimentation rate (ESR), conjugated and unconjugated bilirubin, uraemia and serum creatinine were measured under standardized condition. In particular, 5 mL of blood were drawn from the antecubital vein in heparinized vacuum tubes. Samples collected were centrifuged for 5 minutes at 3000 x g to separate plasma and all plasma samples were stored in multiple aliquots, immediately frozen at -80°C, until assayed within one month from the collection.⁷

Insulin was analyzed by a Cobas^{®} e801 (Roche Diagnostics Italia S.p.a., Monza (MB), Italy) analytical unit which is a high throughput immunochemistry module. Leptin levels were measured by means of enzyme immunoassay (ELISA) kit (cat. No. EH0216; FineTest, Wuhan, China), ghrelin and IGF-1 levels by an enzyme linked immunosorbent assay kit respectively (cat. No. EH0355; FineTest, Wuhan, China) and Human IGF-1 (Insulin-Like Growth Factor 1) by means of an ELISA Kit (cat. No. EH0165; FineTest, Wuhan, China).

All the samples and standards were red by a microplate reader spectrophotometer (Infinite M200, Tecan Group Ltd., Mannedorf, Switzerland).⁷ The Homeostasis Model Assessment of insulin resistance (HOMA-IR), steady state beta cell function (%B) and insulin sensitivity (%S), were calculated from fasting insulin and glucose by means of the HOMA2 Calculator. ^{24,25}

### Anthropometric measures

During the clinical study, the height and body weight were measured twice by the same examiner with a scale (Seca model 700; Seca GmbH, Hamburg, Germany) and stadiometer (Holtain Ltd, UK).^{26,27} During the measurements, subjects wore only underwear. BMI was calculated by dividing the body weight (Kg) by height in meters and was expressed as Kg/m². Waist circumference (WC) was measured twice in a standing position with a non-elastic tape measurement method, while participants were instructed to breathe out mildly, at the midpoint between the top of the iliac crest and the lowest coastal rib.²⁸ An estimation of fat mass (FM, in % and Kg), skeletal muscle mass (MM, in % and Kg) and grade of visceral fat (VF level) was calculated based on and bioelectrical impedance analysis (BIA) devices (Omron HBF-500 BIA, Omron Medizintechnik, Mannheim, Germany).^{54,55}

### Results

102 eligible patients were selected out of 113 patients. In particular, 50 and 52 participants were respectively randomized in FMD4Control group and Control->FMD group (Figure 1, Table 2). Non-adherence to all 6 cycles of the FMD treatment was reported in 16% (n = 8) and 14.8% (n = 7) of FMD4Control and Control→FMD participants, respectively (Figure 1). No differences were found in terms of baseline main parameters when comparing those participants whose health conditions improved with those whose health conditions worsened in terms of olfactory (TDI) and gustatory (TTS) composite score after the 6-month FMD period and in TTS after the control period. On the contrary, participants whose health condions worsened in TDI after the control period were found to have significant lower baseline BMI, weight and WC when compared to those whose health conditions improved in TDI after the same period (more information are reported below). None of participants was found to be hypogeusic, whereas hyposmia was found in 36% (18/50), 10% (4/40) and 13.5% (5/37) FMD4Control participants and in 32% (17/52), 40% (19/47) and 2.6% (1/38) Control→FMD participants, respectively at T0, T1 and T2. Thus, the total number of subjects with hyposmia in Arms 1 and 2 was reduced from 37 out of 97 or 38.1% to 5 out of 78 or 6.4% at the end of the 6 FMD cycles, a 5.9 fold decrease (Figure 2).

**Table 2. Socio-demographic aspects and characteristics of all participants at baseline. Values are given in mean ± standard deviation (SD) and ± 95% confidence interval (CI) for normally distributed variables or median (interquartile range; IQR) for log-normally distributed variables and frequencies n (%) for categorical variables. BMI, body mass index; OSAS, obstructive sleep apnea syndrome; ACEIs, angiotensin-converting enzyme inhibitors; ARBs, angiotensin II receptor blockers; NSAIDs, non-steroidal anti-inflammatory drugs; PPIs, proton-pump inhibitors. OT, odor threshold; OD, odor discrimination; OI, odor identification; and their sum (TDI); TTS, total taste score; ALT, alanine aminotransferase; AST, aspartate aminotransferase; TGs, triglycerides; HDL, high density lipoprotein cholesterol; LDL, low-density lipoprotein cholesterol; ESR, erythrocyte sedimentation rate; CRP, C-reactive protein; HOMA %B, steady state beta cell function; HOMA %S, insulin sensitivity; HOMA-IR, homeostasis model assessment of insulin resistance; WC, waist circumference; BMI, body mass index; ng, nanogram; pg, picogram; ml, milliliter; mg, milligram; dl, deciliter; U, international unit; µU, micro international unit; mm, millimiter; L, liter; h, hour; cm, centimeter; m, meter; Kg, kilogram; %, percentage; FM, fat mass; MM, muscle mass; X², Chi-square. p values < 0.01 were considered significant. *, estimated by means of bioelectrical impedance analysis.**

| | ***FMD*→*Control* (*n* = *50)*** | ***Control→FMD (n = 52)*** | ***X²*/*t-test*** |
|---|---|---|---|
| **Age (years)** | 54.4 ± 12.3 | 52.3 ± 12.1 | p = 0.39 |
| **Gender** | 24 females, 26 males | 29 females, 23 males | X² = 0.61, p = 0.43 |
| **Education** | 14.2 ± 4.2 | 13.6 ± 3.7 | p = 0.45 |

| **Obesity stages** | | | |
|---|---|---|---|
| *Overweight (25≤BMI≤29.9)* | 8(16) | 9 (17.3) | X² = 1.18, p= 0.75 |
| *Stage I obesity* | 25 (50) | 22 (42.3) | |
| *(30≤BMI≤34.9)* | | | |
| *Stage II obesity (35≤BMI≤39.9)* | 12 (24) | 17 (32.6) | |
| *Stage III obesity (BMI≥40)* | 5 (10) | 4 (7.6) | |

| **History of** | | | |
|---|---|---|---|
| *Hypertension* | 29 (58) | 32 (61.5) | X² = 0.03, p = 0.85 |
| *Arthrosis* | 23 (46) | 19 (36.5) | X² = 0.39, p = 0.53 |
| *Thyroid nodules* | 9(18) | 6 (11.5) | X² = 0.63, p = 0.42 |
| *Coronary heart disease* | 7 (14) | 6 (11.5) | X2= 0.1, p = 0.74 |
| *Type 2 diabetes* | 21 (42) | 22 (42.3) | X2 = 0.004, p = 0.98 |
| *Psoriasis* | 1 (2) | 0 (0) | - |
| *OSAS* | 13 (26) | 15 (28.8) | X² = 0.05, p = 0.8 |

| **Medication** | | | |
|---|---|---|---|
| *Metformin* | 21 (42) | 22 (42.3) | X2 = 0.004, p = 0.98 |
| *ACEIs*/*ARBs* | 20 (40) | 24 (46.1) | X² = 0.15, p = 0.69 |
| *Loop diuretics* | 6 (12) | 5 (9.6) | X² = 0.12, p = |
| | | | 0.72 |
| *Calcium antagonists* | 13 (26) | 14 (26.9) | X² = 0.0065, p = 0.93 |
| *Thiazide diuretics* | 7 (14) | 9 (17.3) | X² = 0.15, p = 0.69 |
| *NSAIDs* | 23 (46) | 25 (48) | X² = 0.01, p = 0.89 |
| *PPIs* | 26 (52) | 24 (46.1) | X² = 0.11, p = 0.72 |
| *Statin* | 18 (36) | 16 (30.7) | X² = 0.15, p = 0.69 |

| ***Chemosensory testing*** | | | |
|---|---|---|---|
| **OT** | 7.49 ± 2.74 (6.73 - 8.25) | 7.25 ± 1.69 (6.8 - 7.71) | p = 0.6 |
| **OD** | 11.28 ± 1.73 (10.79 - 11.76) | 11.71 ± 2.12 (11.13 - 12.28) | p = 0.26 |
| **OI** | 12.58 ± 1.59(12.13 - 13.02) | 12.78 ± 1.14 (12.47 - 13.09) | p = 0.44 |
| **TDI** | 31.25 ±3.74(30.21 - 32.29) | 31.75 ± 3.42 (30.82 - 32.69) | p = 0.47 |
| **Sweet** | 6.64 ± 1.28 (6.28 - 6.99) | 6.71 ± 1.31 (6.35 - 7.06) | p = 0.7 |
| **Sour** | 4.9 ± 1.65 | 4.88 ± 1.26 (4.54 - | p = 0.95 |
| | (4.44 - 5.35) | 5.22) | |
| **Salty** | 5.78 ± 1.56 (5.34 - 6.21) | 5.69 ± 1.65 (5.24 - 6.14) | p = 0.78 |
| **Bitter** | 5.62 ± 1.57 (5.18 - 6.05) | 5.73 ± 1.72 (5.26 - 6.2) | p = 0.73 |
| **TTS** | 22.94 ± 4.1 (21.8 - 24.07) | 23.01 ± 3.97 (21.93 - 24.1) | p = 0.92 |

| ***Biochemical Assays*** | | | |
|---|---|---|---|
| **Leptin (ng/ml)** | 26.89 (7.86) | 26.31 (14.74) | p = 0.28 |
| **Ghrelin (pg/ml)** | 197.13 ± 42.13 (185.45 208.8) | 203.86 ± 37.74 (193.6 - 214.12) | p = 0.39 |
| **IGF-1 (ng/ml)** | 159.51 ± 23.75 (152.92 -166.09) | 157.06 ± 26.38 (149.89 - 164.23) | p = 0.62 |
| **Serum glucose (mg/dl)** | 99 (13.75) | 99 (22.25) | p = 0.59 |
| **Insulin (µU/ml)** | 11.49 (7.51) | 10.61 (7.1) | p = 0.25 |
| **Total cholesterol (mg/dl)** | 223.28 ± 38.36 (212.64 -233.91) | 219.59 ± 36.3 (209.72 - 229.46) | p = 0.61 |
| **LDL (mg/dl)** | 142.32 ± 37.93 (131.8-152.83) | 40.78 ± 35.27 (131.2 - 150.37) | p = 0.83 |
| **HDL (mg/dl)** | 55 (22.759) | 52 (19.25) | p = 0.4 |
| **TGs (mg/dl)** | 111 (76.25) | 115 (41.25) | p = 0.69 |
| **Conjugated bilirubin (mg/dl)** | 0.16 ± 0.06 (0.14 - 0.18) | 0.18 ± 0.11 (0.15 - 0.21) | p = 0.22 |
| **Unconjugated bilirubin (mg/dl)** | 0.42 ± 0.19 (0.37 - 0.48) | 0.42 ± 0.22 (0.36 - 0.48) | p = 0.97 |
| **ESR (mm/h)** | 12 (11.75) | 11 (10.25) | p = 0.73 |
| **CRP (mg/L)** | 0.9 (2.69) | 1.76 (2.62) | p = 0.79 |
| **AST (U/L)** | 23.32 ± 8.42 (20.98 - 25.65) | 23.25 ± 7.18 (21.29 - 25.2) | p = 0.96 |
| **ALT (U/L)** | 25.72 ± 11.85 (22.43 - 29) | 28.32 ± 13.56 (24.64 - 32.01) | p = 0.83 |
| **Uraemia (mg/dl)** | 35.36 ± 12.78 (31.81 - 38.9) | 35.17 ± 9.29 (32.64 - 37.69) | p = 0.93 |
| **Serum creatinine (mg/dl)** | 0.84 ± 0.15 (0.8 - 0.88) | 0.84 ± 0.15 (0.8 - 0.89) | p = 0.95 |
| **HOMA %B** | 102.97± 34.11 (93.51 - 112.42) | 110.95 ± 45.34 (98.62 - 123.27) | p = 0.31 |
| **HOMA %S** | 76.68± 34.65 (67.07 -86.28) | 76.15 ± 40.96 (65.01 - 87.28) | p = 0.94 |
| **HOMA IR** | 1.56± 0.65 (1.38 -1.74) | 1.71 ± 0.96 (1.45 - 1.97) | p = 0.36 |

| ***Anthropometric Variables*** | | | |
|---|---|---|---|
| **WC (cm)** | 111.68 ± 10.38 (108.8 - | 112.18 ± 9.6 (109.57 - 114.79) | p = 0.8 |
| | 114.55) | | |
| **Weight (kg)** | 94.57 ± 14.5 (90.54 - 98.59) | 97.07 ± 14.44 (93.15 - 101) | p = 0.38 |
| **BMI (Kg/m²)** | 33.7 ± 4.38 (32.48 - 34.91) | 33.87 ± 3.91 (32.8 - 34.93) | p = 0.83 |
| **FM%*** | 39.72 ± 8.25 (37.43 - 42.01) | 40.95 ± 7.32 (38.96 - 42.94) | p = 0.42 |
| **FM (Kg)*** | 37.41 ± 9.47 (34.78 - 40.04) | 39.7 ± 9.11 (37.22 - 42.18) | p = 0.21 |
| **MM%*** | 26.51 ± 4.33 (25.31 - 27.71) | 25.9 ± 4.24 (24.75 - 27.05) | p = 0.47 |
| **MM (Kg)*** | 25.16 ± 5.9 (23.53 - 26.8) | 25.22 ± 5.86 (23.62 - 26.81) | p = 0.96 |
| **VFLevel*** | 15.38 ± 4.93 (14.01 - 16.74) | 14.4 ± 4.68 (13.13 - 15.67) | p = 0.3 |

### Changes in chemosensory testing:

### Changes from baseline

After the 6 FMD cycles, the within-subject analysis found in FMD4Control participants (n = 40) showed a significant increase in odor threshold (OT), odor discrimination (OD), TDI, TTS and sweet (Table 3, Figure 3). As expected, no significant differences were found in Control→FMD participants (n = 47) when comparing the results at T0 with the results at T1 (routinary eating period). On the contrary, when the results at T2 are compared with the results at T1 (number of patients = 38), the impact of 6 FMD cycles lead to a significant increase in OT, OD, TDI, TTS, sweet and sour (Table 4, Figure 3) for the Control→FMD group, similar to FMD→Control group at T1 vs. T0. A significant increase in ΔOT, ΔOD, Δodoro identification (OI), ΔTDI, Δsweet, Δsour, Δbitter, Δsalty and ΔTTS were found in all the participants after 6 FMD component administrations (FMD4Control + Control->FMD) compared to Control->FMD participants during the first 6-month period (routinary eating) (Table 3, Figure 2).

In agreement with the lack of differences for many markers between groups at T2, some of chemosensory testing variables were found to be significantly different when also comparing FMD4Control group at T2 with FMD4Control group at T0 (n = 37) values: TDI (p = 0.001), TTS (p = 0.004), sweet (p < 0.001) whereas no significant differences were found in OT (p = 0.017), OD (p = 0.015) and OI (p = 0.19), sour (p = 0.019), salty (p = 0.34), bitter (p = 0.1). At T2, after FMD4Control participants returned to their routinary eating for 6 months and no significant differences were found in OT (p = 0.66), OD (p = 0.86), OI (p = 0.94), TDI (p = 0.69), sweet (p = 0.79), sour (p = 0.92), bitter (p = 0.23), salty (p = 0.54), TTS (p = 0.73), in particular when compared to the results at T1.

When comparing patients of Control-^FMD at T2 with said patients of Control->FMD at T0, a similar behavior was found, showing a significant increase in OT (p < 0.001), OD (p = 0.002), TDI (p < 0.001), TTS (p < 0.001), sweet (p < 0.001) and sour (p < 0.001).

| | | **Baseline** | **TRL: 6 months after baseline / FMD: 5 days after sixth FMD cycle** | | | |
|---|---|---|---|---|---|---|
| | | **Mean ± SD (CI)/median (IQR)** | **Mean ± SD (CI)/median (IQR)** | **p** 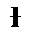 | **Difference: Δ Mean ±SD** | **Efficacy (comparing Δ)§ p** |
| Hyposmic patients | MD 1 | 8/50 (36%) | 4/40 (10/%) | | | |
| | MD 2 | 19/47 (40%) | 1/38 (2.6%) | | | |
| | Control Diet, Control->FMD | 17/52 (32%) | 19/47 (40%) | | | |
| ***OT*** | FMD 1 (n = 40) | 7.58 ± 2.42 (6.83 - 8.33) | 8.98 ± 2.03 (8.35 - 9.61) | 0.006 | 1.45 ± 1.08 | < 0.001 |
| | FMD 2 (n = 38) | 7.11 ± 1.48 (6.64 -7.58) | 8.63 ± 1.71 (8.08 - 9.17) | < 0.001 | | |
| | Control Diet, Control→FMD (n = 47) | 7.15 ± 1.65 (6.68 - 7.63) | 7.06 ± 1.49 (6.63 - 7.49) | 0.76 | -0.09 ± 0.56 | |
| ***OD*** | FMD 1 (n = 40) | 10.97 ± 1.6 (10.47 - 11.47) | 11.97 ± 1.64 (11.46 - 12.48) | 0.007 | 1.06 ± 1.2 | < 0.001 |
| | FMD 2 (n = 38) | 11.73 ± 1.81 (11.16 - 12.31) | 12.86 ± 1.39 (12.42 - 13.31) | 0.003 | | |
| | Control Diet, Control→FMD (n = 47) | 11.55 ± 2.16 (10.93 - 12.17) | 11.57 ± 1.82 (11.05 - 12.09) | 0.95 | 0.02 ± 0.92 | |
| ***OI*** | FMD 1 (n = 40) | 12.75 ± 1.62 (12.24 - 13.25) | 13.25 ± 1.62 (12.74 - 13.75) | 0.17 | 0.51 ± 0.92 | < 0.001 |
| | FMD 2 (n = 38) | 12.65 ± 1.25 (12.25 - 13.05) | 13.18 ± 1.22 (12.79 - 13.57) | 0.06 | | |
| | Control Diet, Control→FMD (n = 47) | 12.82 ± 1.1 (12.51 - 13.14) | 12.48 ± 1.23 (12.13 - 12.84) | 0.16 | -0.34 ± 0.93 | |
| ***TDI*** | FMD 1 (n = 40) | 31.18 ± 3.6 (30.06 - 32.29) | 34.25 ± 3.46 (33.17 - 35.32) | < 0.001 | 3.11 ± 2.37 | < 0.001 |
| | FMD 2 (n = 38) | 31.51 ± 2.86 (30.6 - 32.42) | 34.68 ± 2.86 (33.77 - 35.59) | < 0.001 | | |
| | Control Diet, Control→FMD (n = 47) | 31.54 ± 3.45 (30.55 - 32.52) | 31.12 ± 2.98 (30.27 - 31.98) | 0.53 | -0.41 ± 1.42 | |
| ***Sweet*** | FMD 1 (n = 40) | 6.52 ± 1.32 (6.11 - 6.93) | 7.37 ± 0.89 (7.09 - 7.65) | 0.001 | 0.91 ± 0.98 | < 0.001 |
| | FMD 2 (n = 38) | 6.63 ± 0.94 (6.33 - 6.93) | 7.6 ± 0.78 (7.35 - 7.85) | < 0.001 | | |
| | Control Diet, Control→FMD (n = 47) | 6.8 ± 1.32 (6.42 - 7.18) | 6.63 ± 1 (6.34 - 6.92) | 0.48 | -0.17 ± 0.81 | |
| ***Sour*** | FMD 1 (n = 40) | 4.85 ± 1.59 (4.35 - 5.34) | 5.67 ± 1.28 (5.27 - 6.07) | 0.012 | 0.88 ± 0.95 | < 0.001 |
| | FMD 2 (n = 38) | 5.02 ± 1.02 (4.69 - 5.35) | 5.97 ± 1.05 (5.63 - 6.3) | < 0.001 | | |
| | Control Diet, Control→FMD (n = 47) | 4.8 ± 1.26 (4.44 - 5.16) | 5.06 ± 1.09 (4.75 - 5.37) | 0.29 | 0.25 ± 0.67 | |
| ***Salty*** | FMD 1 (n = 40) | 5.65 ± 1.4 (5.21 - 6.08) | 6.17 ± 1.37 (5.74 - 6.6) | 0.09 | 0.52 ± 1.07 | 0.004 |
| | FMD 2 (n = 38) | 5.52 ± 1.53 (5.03 - 6.01) | 6.05 ± 1.33 (5.62 - 6.47) | 0.11 | | |
| | Control Diet, Control→FMD (n = 47) | 5.68 ± 1.6 (5.22 - 6.13) | 5.65 ± 1.56 (5.21 - 6.1) | 0.94 | -0.02 ±0.89 | |
| ***Bitter*** | FMD 1 (n = 40) | 5.52 ± 1.53 (5.04 - 6) | 6.32 ± 1.28 (5.92 - 6.72) | 0.013 | 0.8 ± 1.05 | < 0.001 |
| | FMD 2 (n = 38) | 5.5 t 1.57 (5 - 5.99) | 6.31 ± 1.33 (5.89 - 6.74) | 0.017 | | |
| | Control Diet, Control→FMD (n = 47) | 5.72 ± 1.72 (5.22 - 6.21) | 5.59 ± 1.59 (5.13 - 6.05) | 0.71 | -0.12 ± 0.84 | |
| ***TTS*** | FMD 1 (n = 40) | 22.55 ± 4.08 (21.28 - 23.81) | 25.27 ± 4.06 (24.01 - 26.53) | 0.003 | 2.98 ± 2.92 | < 0.001 |
| | FMD 2 (n = 38) | 22.68 ± 3.63 (21.52 - 23.84 | 25.94 ± 3.52 (24.82 - 27.06) | < 0.001 | | |
| | Control Diet, Control→FMD (n = 47) | 23.02 ± 4.14 (21.83 - 24.2) | 22.95 ± 3.75 (21.88 - 24.03) | 0.93 | -0.06 ± 1.6 | |
| ***Leptin (ng*/*ml)*** | FMD 1 (n = 40) | 26.02 (7.17) | 18.19 (12.3) | < 0.001 | -8.88 ± 5.38 | < 0.001 |
| | FMD 2 (n = 38) | 30.86 (13.61) | 21.38 (8.44) | < 0.001 | | |
| | Control Diet, Control→FMD (n = 47) | 26.95 (15) | 28.56 (14.16) | 0.89 | 0.33 ± 3.16 | |
| ***Ghrelin (pg*/*ml)*** | FMD 1 (n = 40) | 199.83 ± 43.03 | 251.9 ± 52.31 (235.69 - | < 0.001 | 47.2 ± 35.67 | < 0.001 |
| | | (186.49 - 213.16) | 268.12) | | | |
| | FMD 2 (n = 38) | 208.74 ± 40.75 (195.78 - 221.7) | 250.83 ± 50.28 (234.84 - 266.82) | < 0.001 | | |
| | Control Diet, Control→FMD (n = 47) | 204.46 ± 38.98 (193.31 - 215.6) | 207.28 ± 37.82 (196.47 - 218.1) | 0.72 | 2.82 ± 9.21 | |
| ***IGF-1 (ng*/*ml)*** | FMD 1 (n = 40) | 162.11 ± 24.97 (154.37 - 169.85) | 144.46 ± 27.01 (136.09 - 152.83) | 0.003 | -16.93 ± 20.31 | < 0.001 |
| | FMD 2 (n = 38) | 156.96 ± 24.44 (149.19 - 164.74) | 140.78 ± 23.7 (133.24 - 148.32) | 0.004 | | |
| | Control Diet, Control→FMD (n = 47) | 154.3 ± 26.04 (146.85 - 161.75) | 156.82 ± 24.87 (149.7 - 163.93) | 0.63 | 2.51 ± 8.67 | |
| ***Serum glucose (mg*/*dl)*** | FMD 1 (n = 40) | 99.5 (13.5) | 90 (12) | 0.008 | -10.26 ± 8.4 | < 0.001 |
| | FMD 2 (n = 38) | 96 (19.75) | 87 (14.75) | < 0.001 | | |
| | Control Diet, Control→FMD (n = 47) | 96 (21.5) | 98 (19.5) | 0.71 | 1.02 ± 3.61 | |
| ***Insulin (µU*/*ml)*** | FMD 1 (n = 40) | 11.99 (7.87) | 7.95 (5.4) | < 0.001 | -4.26 ± 3.88 | < 0.001 |
| | FMD 2 (n = 38) | 12.13 (9.28) | 8.96 (6.01) | 0.002 | | |
| | Control Diet, Control→FMD (n = 47) | 11 (7.47) | 11.4 ±1 (7.26) | 0.99 | 0 ± 1.25 | |
| ***Total cholesterol (mg*/*dl)*** | FMD 1 (n = 40) | 227.15 ± 37.89 (215.4 - 238.89) | 195.6 ± 31.19 (185.93 - 205.26) | < 0.001 | -31.75 ± 20.34 | < 0.001 |
| | FMD 2 (n = 38) | 214.13 ± 33.6 (203.44 - 224.81) | 182.15 ± 30.09 (172.58 - 191.72) | < 0.001 | | |
| | Control Diet, Control→FMD (n = 47) | 218.85 ± 37.86 (208.02 - 229.67) | 217.51 ± 33.44 (207.94 - 227.07) | 0.85 | -1.34 ± 19.97 | |
| ***LDL (mg*/*dl)*** | FMD 1 (n = 40) | 145.4 ± 33.19 (135.11 - 155.68) | 123.05 ± 24.48 (115.46-130.63) | < 0.001 | -27.38 ± 19.27 | < 0.001 |
| | FMD 2 (n = 38) | 132.89 ± 33.23 (122.32 - 143.46) | 100.21 ± 29.78 (90.74 - 109.68) | < 0.001 | | |
| | Control Diet, Control→FMD (n = 47) | 139.74 ± 36.62 (129.27 - 150.21) | 138.93 ± 33.33 (129.4 - 148.46) | 0.91 | - 0.8 ± 14.26 | |
| ***HDL (mg*/*dl)*** | FMD 1 (n = 40) | 54.5 (24.75) | 57 (15.25) | 0.94 | 3.85 ± | 0.01 |
| | FMD 2 (n = 38) | 52 (19.25) | 60 (15) | 0.002 | 11.69 | |
| | Control Diet, Control→FMD (n = 47) | 51 (19.5) | 52 (19) | 0.58 | -1.4 ± 9.43 | |
| ***TGs (mg*/*dl)*** | FMD 1 (n = 40) | 115 (83.5) | 91 (49.25) | 0.03 | -24.62 ± 29.55 | < 0.001 |
| | FMD 2 (n = 38) | 113.5 (56.25) | 92.5 (55.5) | 0.03 | | |
| | Control Diet, Control→FMD (n = 47) | 114 (44) | 114 (50.5) | 0.72 | -4.17 ± 23.06 | |
| ***Conjugated bilirubin (mg*/*dl)*** | FMD 1 (n = 40) | 0.16 ± 0.06 (0.13 - 0.18) | 0.17 ± 0.08 (0.14 - 0.2) | 0.37 | -0.01 ± 0.09 | 0.09 |
| | FMD 2 (n = 38) | 0.21 ± 0.12 (0.17 - 0.25) | 0.17 ± 0.08 (0.14 - 0.2) | 0.09 | | |
| | Control Diet, Control→FMD (n = 47) | 0.18 ± 0.11 (0.15 - 0.22) | 0.2 ± 0.11 (0.17 - 0.23) | 0.52 | 0.01 ± 0.06 | |
| ***Unconjugated bilirubin (mg*/*dl)*** | FMD 1 (n = 40) | 0.42 ± 0.18 (0.36 - 0.47) | 0.44 ± 0.2 (0.37 -0.5) | 0.63 | -0.01 ± 0.15 | 0.16 |
| | FMD 2 (n = 38) | 0.47 ± 0.21 (0.4 - 0.54) | 0.42 ± 0.19 (0.36 - 0.49) | 0.36 | | |
| | Control Diet, Control→FMD (n = 47) | 0.43 ± 0.23 (0.36 - 0.49) | 0.45 ± 0.19 (0.4 - 0.51) | 0.56 | 0.02 ± 0.12 | |
| ***ESR (mm*/*h)*** | FMD 1 (n = 40) | 12 (11.25) | 7 (7.25) | 0.04 | -4.02 ± 4.31 | < 0.001 |
| | FMD 2 (n = 38) | 13 (13.25) | 7 (9) | 0.018 | | |
| | Control Diet, Control→FMD (n = 47) | 11 (11) | 13 (10.5) | 0.88 | 0.25 ± 1.51 | |
| ***CRP (mg*/*L)*** | FMD 1 (n = 40) | 0.9 (2.48) | 0.5 (1.23) | 0.04 | -0.73 ± 1.64 | < 0.001 |
| | FMD 2 (n = 38) | 1.56 (2.51) | 1.07 (1.47) | 0.3 | | |
| | Control Diet, Control->FMD (n = 47) | 1.75 (2.65) | 1.7 (2.4) | 0.78 | 0.15 ± 0.43 | |
| ***AST (U*/*L)*** | FMD 1 (n = 40) | 23.75 ± 9.02 (20.95 - 26.54) | 19.25 ± 5.9 (17.41-21.08) | 0.01 | -4.67 ± 5.37 | < 0.001 |
| | FMD 2 (n = 38) | 23.55 ± 7.52 (21.15 - 25.94) | 18.68 ± 5.88 (16.81 - 20.55) | 0.002 | | |
| | Control Diet, Control→FMD (n = 47) | 23.53 ± 7.45 (21.4 - 25.66) | 23.57 ± 6.87 (21.61 - 25.53) | 0.97 | 0.04 ± 2.06 | |
| ***ALT (U*/*L)*** | FMD 1 (n = 40) | 26.82 ± 12.89 (22.82 - 30.82) | 20.1 ± 9.91 (17.02 - 23.17) | 0.01 | -7.32 ± 8.97 | < 0.001 |
| | FMD 2 (n = 38) | 29.05 ± 15.48 (24.13 - 33.97) | 21.1 ± 10.13 (17.88 - 24.32) | 0.009 | | |
| | Control Diet, Control→FMD (n = 47) | 28.4 ± 14.17 (24.35 - 32.45) | 28.8 ± 14.09 (24.78 - 32.83) | 0.89 | 0.4 ± 3.1 | |
| ***Uraemia (mg*/*dl)*** | FMD 1 (n = 40) | 35.97 ± 13.19 (31.88 - 40.05) | 32.33 ± 9.89 (29.26 - 35.39) | 0.16 | -4.66 ± 7.78 | 0.003 |
| | FMD 2 (n = 38) | 33.6 ± 8.19 | 27.86 ± 6.21 | < | | |
| | | (31 - 36.2) | (25.89 - 29.84) | 0.001 | | |
| | Control Diet, Control→FMD (n = 47) | 35.18 ± 9.67 (32.42 - 37.95) | 34.11 ± 7.95 (31.84 - 36.39) | 0.55 | -1.07 ± 3.75 | |
| ***Serum creatinine (mg*/*dl)*** | FMD 1 (n = 40) | 0.85 ± 0.15 (0.81 - 0.9) | 0.82 ± 0.15 (0.77 - 0.87) | 0.33 | -0.04 ± 0.09 | < 0.001 |
| | FMD 2 (n = 38) | 0.88 ± 0.17 (0.82 - 0.93) | 0.82 ± 0.15 (0.77 - 0.87) | 0.16 | | |
| | Control Diet, Control→FMD (n = 47) | 0.85 ± 0.15 (0.8 - 0.89) | 0.87 ± 0.16 (0.82 - 0.92) | 0.53 | 0.02 ± 0.11 | |
| ***HOMA %B*** | FMD 1 (n = 40) | 105.72 ± 37.15 (94.2 - 117.23) | 96.1 ± 34.03 (85.55 - 106.65) | 0.23 | -6.23 ± 24.56 | 0.13 |
| | FMD 2 (n = 38) | 116.11 ± 47.42 (101.03 - 131.19) | 113.44 ± 44.23 (99.37 - 127.5) | 0.79 | | |
| | Control Diet, Control→FMD (n = 47) | 114.64 ± 45.66 (101.58 - 127.69) | 114.15 ± 44.53 (101.42 - 126.88) | 0.95 | -0.46 ± 12.72 | |
| ***HOMA %S*** | FMD 1 (n = 40) | 74.5 ± 34.79 (63.72 - 85.28) | 113.88 ± 54.92 (96.86 - 130.9) | < 0.001 | 36.04 ± 32.08 | < 0.001 |
| | FMD 2 (n = 38) | 69.65 ± 38.45 (57.42 - 81.88) | 102.18 ± 59.49 (83.27 - 121.1) | 0.005 | | |
| | Control Diet, Control→FMD (n = 47) | 75.02 ± 42.83 (62.77 - 87.26) | 69.78 ± 35.29 (59.69 - 79.87) | 0.51 | -5.24 ± 24.08 | |
| ***HOMA IR*** | FMD 1 (n = 40) | 1.6 ± 0.63 (1.4 - 1.8) | 1.07 ± 0.45 (0.92 - 1.21) | < 0.001 | -0.56 ± 0.52 | < 0.001 |
| | FMD 2 (n = 38) | 1.87 ± 1 (1.55 - 2.19) | 1.28 ± 0.63 (1.08 - 1.48) | 0.002 | | |
| | Control DietControl→FMD (n = 47) | 1.77± 0.99 (1.48 - 2.05) | 1.8 ± 0.92 (1.54 - 2.06) | 0.86 | 0.03 ± 0.26 | |
| ***WC (cm)*** | FMD 1 (n = 40) | 110.34 ± 9.21 (107.49 - 113.19) | 103.27 ± 9.78 (100.24 - 106.31) | 0.001 | -7.03 ± 4.53 | < 0.001 |
| | FMD 2 (n = 38) | 114.59 ± 8.63 (111.84 - 117.33) | 107.59 ± 8.99 (104.73 - 110.45) | < 0.001 | | |
| | Control Diet, Control→FMD (n = 47) | 112.53 ± 9.33 (109.86 - 115.2) | 113.09 ± 8.98 (110.52 - 115.66) | 0.76 | 0.55 ± 1.76 | |
| ***Weight (kg)*** | FMD 1 (n = 40) | 92.81 ± 12.65 (88.89 - 96.74) | 86.16 ± 12.68 (82.23 - 90.09) | 0.02 | -6.44 ± 2.83 | < 0.001 |
| | FMD 2 (n = 38) | 98.87 ± 14.78 (94.17 -103.57) | 92.63 ± 14.68 (87.97 - 97.3) | 0.06 | | |
| | Control Diet, Control→FMD (n = 47) | 96.94 ± 14.21 (92.87 - 101) | 97.07 ± 14.29 (92.98 - 101.16) | 0.96 | 0.13 ± 2.16 | |
| ***BMI (Kg*/*m²)*** | FMD 1 (n = 40) | 33.36 ± 4.25 (32.04 - 34.68) | 30.89 ± 4.24 (29.57 - 32.2) | 0.011 | -2.33 ± 1.1 | < 0.001 |
| | FMD 2 (n = 38) | 34.34 ± 3.35 (33.27 - 35.4) | 32.14 ± 3.43 (31.05 - 33.24) | 0.006 | | |
| | Control Diet, Control→FMD (n = 47) | 33.85 ± 3.56 (32.83 - 34.87) | 33.88 ± 3.44 (32.9 - 34.87) | 0.96 | 0.03 ± 0.85 | |
| ***FM%**** | FMD 1 (n = 40) | 39.52 ± 8.39 (36.91 - 42.12) | 28.1 ± 5.32 (26.44 - 29.75) | < 0.001 | -9.56 ± 8.17 | < 0.001 |
| | FMD 2 (n = 38) | 40.78 ± 6.72 (38.64 - 42.92) | 33.17 ± 5.76 (31.34 - 35.01) | < 0.001 | | |
| | Control Diet, Control→FMD (n = 47) | 40.88 ± 7.21 (38.82 - 42.94) | 40.87 ± 7.02 (38.86 - 42.88) | 0.99 | 0 ± 1.8 | |
| ***FM (Kg)**** | FMD 1 (n = 40) | 36.52 ± 8.94 (33.74 - 39.29) | 25.08 ± 7.59 (22.72 - 27.43) | < 0.001 | -10.47 ± 8.45 | < 0.001 |
| | FMD 2 (n = 38) | 40.12 ± 8.08 (37.55 - 42.69) | 30.65 ± 6.76 (28.5 - 32.8) | < 0.001 | | |
| | Control Diet, Control→FMD (n = 47) | 39.5 ± 8.54 (37.06 - 41.94) | 39.53 ± 8.32 (37.15 - 41.91) | 0.98 | 0.03 ± 2.36 | |
| ***MM*%*** | FMD 1 (n = 40) | 26.53 ± 4.46 (25.14 - 27.91) | 36.62 ± 7.67 (34.24 - 39) | < 0.001 | 8.52 ± 8.48 | < 0.001 |
| | FMD 2 (n = 38) | 26.33 ± 4.31 (24.96 - 27.7) | 33.2 ± 4.71 (31.7 - 34.7) | 0.001 | | |
| | Control Diet, Control→FMD (n = 47) | 25.95 ± 4.24 (24.74 - 27.17) | 26.24 ± 4.32 (25 -27.47) | 0.74 | 0.28 ± 2.18 | |
| ***MM (Kg)**** | FMD 1 (n = 40) | 24.71 ± 5.66 (22.95 - 26.47) | 32.48 ± 8.48 (29.85-35.11) | < 0.001 | 6.2 ± 7.9 | < 0.001 |
| | FMD 2 (n = 38) | 26.15 ± 6.32 (24.14 - 28.16) | 30.72 ± 6.07 (28.79 - 32.65) | 0.002 | | |
| | Control Diet, Control→FMD (n = 47) | 25.26 ± 5.98 (23.55 - 26.97) | 25.56 ± 6.07 (23.82 - 27.29) | 0.81 | 0.29 ± 2.29 | |
| ***VFlevel**** | FMD 1 (n = 40) | 15.12 ± 4.43 (13.75 - 16.49) | 12.22 ± 4.13 (10.94 - 13.5) | 0.003 | -3.02 ± 1.77 | < 0.001 |
| | FMD 2 (n = 38) | 15.1 ± 4.42 (13.6916.51) | 11.94 ± 4.21 (10.6 - 13.28) | 0.002 | | |
| | Control Diet, Control→FMD (n = 47) | 14.36 ± 4.72 (13.01 - 15.71) | 14.4 ± 4.64 (13.07 - 15.73) | 0.96 | 0.04 ± 0.83 | |

*Table 3.* Changes from baseline in chemosensory testing, biochemical assays and anthropometric measures in subjects who completed the trial. FMD 1, FMD4Control; FMD 2, Control->FMD. OT, odor threshold; OD, odor discrimination; OI, odor identification; and their sum (TDI); TTS, total taste score; ALT, alanine aminotransferase; AST, aspartate aminotransferase; TGs, triglycerides; HDL, high density lipoprotein cholesterol; LDL, low-density lipoprotein cholesterol; ESR, erythrocyte sedimentation rate; CRP, C-reactive protein; HOMA %B, steady state beta cell function; HOMA %S, insulin sensitivity; HOMA-IR, homeostasis model assessment of insulin resistance; WC, waist circumference; BMI, body mass index; ng, nanogram; pg, picogram; ml, milliliter; mg, milligram; dl, deciliter; U, international unit; µU, micro international unit; mm, millimiter; L, liter; h, hour; cm, centimeter; m, meter; Kg, kilogram; %, percentage; FM, fat mass; MM, muscle mass; VF, visceral fat. *, estimated by means of bioelectrical impedance analysis. Values are given in mean ± standard deviation (SD) and ± 95% confidence interval (CI) for normally distributed variables or median (interquartile range; IQR) for log-normally distributed variables. 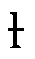 p values comparing within-group changes were calculated using paired two-tailed Student's t test (p values < 0.01 were considered significant). Comparisons between the combination of pre-post FMD differences (Δ) in FMD 1 and FMD 2 (n = 78) participants and Control->FMD T0-T1 (Control Diet) Δ values (n = 47) participants performed using two-tailed two-sample t-tests (p values < 0.01 were considered significant).

### Between-group analysis

The between-group analysis showed that - after 6 FMD cycles - FMD4Control participants exhibited a significant increase in OT (p < 0.001), OI (p = 0.015), and TDI (p < 0.001) as well as in sweet (p < 0.001), and TTS (p = 0.007) when compared with Control->FMD participants. No significant between-group changes were found in OD (p = 0.28) and salty (p = 0.1), although an increase taste perception was observed for sour (p = 0.018) and bitter (p = 0.02) (Table 4, Figure 4). After the second 6-month period during which Control→FMD participants underwent the 6 FMD cycles and FMD4Control participants followed their routinary eating but discontinued FMD cycles only OD was found to be significantly higher in Control→FMD (12.86 ± 1.39) compared to FMD4Control (11.87 ± 1.83) participants (p = 0.01). Said results indicate that the effects of FMD cycles in FMD4Control are long-lasting.

**Table 4. Between-group comparisons in chemosensory testing, biochemical assays and anthropometric measures in all participants at T0, T1 and T2: OT, odor threshold; OD, odor discrimination; OI, odor identification; and their sum (TDI); TTS, total taste score; ALT, alanine aminotransferase; AST, aspartate aminotransferase; TGs, triglycerides; HDL, high density lipoprotein cholesterol; LDL, low-density lipoprotein cholesterol; ESR, erythrocyte sedimentation rate; CRP, C-reactive protein; HOMA %B, steady state beta cell function; HOMA %S, insulin sensitivity; HOMA-IR, homeostasis model assessment of insulin resistance; WC, waist circumference; BMI, body mass index; ng, nanogram; pg, picogram; ml, milliliter; mg, milligram; dl, deciliter; U, international unit; µU, micro international unit; mm, millimiter; L, liter; h, hour; cm, centimeter; m, meter; Kg, kilogram; %, percentage; FM, fat mass; MM, muscle mass; VF, visceral fat. *, estimated by means of bioelectrical impedance analysis. Values are given in mean ± standard deviation (SD) and ± 95% confidence interval (CI) for normally distributed variables or median (interquartile range; IQR) for log-normally distributed variables.**

| | **T0** | | **p** | **T1** | | **p** | **T2** | | **p** |
|---|---|---|---|---|---|---|---|---|---|
| | **FMD->Control (n = 50)** | ***Control->FMD (n* = *52)*** | | **FMD->Control (n = 40)** | **Control->FMD (n = 47)** | | **FMD->Control (n = 37)** | **Control->FMD (n = 38)** | |
| | **Mean ± SD (CI)/median (IQR)** | **Mean ± SD (CI)/median (IQR)** | | **Mean ± SD (CI)/median (IQR)** | **Mean ± SD (CI)/median (IQR)** | | **Mean ± SD (CI)/median (IQR)** | **Mean ± SD (CI)/median (IQR)** | |
| | ***Chemosensory testing*** | | | | | | | | |
| **OT** | 7.49 ± 2.74 (6.73 - 8.25) | 7.25 ± 1.69 (6.8 - 7.71) | 0.6 | 8.98 ± 2.03 (8.35 - 9.61) | 7.06 ± 1.49 (6.63 - 7.49) | < 0.001 | 8.83 ± 2.06 (8.17 - 9.5) | 8.63 ± 1.71 (8.08 - 9.17) | 0.63 |
| **OD** | 11.28 ± 1.73 (10.79 - 11.76) | 11.71 ± 2.12 (11.13-12.28) | 0.26 | 11.97 ± 1.64 (11.46 - 12.48) | 11.57 ± 1.82(11.05 - 12.09) | 0.28 | 11.87 ± 1.83 (11.28 -12.46) | 12.86 ± 1.39 (12.42-13.31) | 0.01 |
| **OI** | 12.58± 1.59(12.13 - 13.02) | 12.78 ± 1.14(12.47-13.09) | 0.44 | 13.25 ± 1.62 (12.74-13.75) | 12.48 ± 1.23 (12.13 - 12.84) | 0.015 | 13.27 ± 1.5(12.78 - 13.75) | 13.18 ± 1.22 (12.79 - 13.57) | 0.78 |
| **TDI** | 31.25 ±3.74(30.21 - 32.29) | 31.75 ± 3.42(30.82 - 32.69) | 0.47 | 34.25 ± 3.46 (33.17 - 35.32) | 31.12 ± 2.98 (30.27 - 31.98) | < 0.001 | 33.98 ± 3.86 (32.74 - 35.23) | 34.68 ± 2.86 (33.77 - 35.59) | 0.37 |
| **Sweet** | 6.64 ± 1.28 (6.28 - 6.99) | 6.71 ± 1.31 (6.35 - 7.06) | 0.7 | 7.37 ± 0.89 (7.09 - 7.65) | 6.63 ± 1 (6.34 - 6.92) | < 0.001 | 7.43 ± 0.83 (7.16 - 7.7) | 1.6 ± 0.78 (7.35 - 7.85) | 0.35 |
| **Sour** | 4.9 ± 1.65 (4.44 - 5.35) | 4.88 ± 1.26 (4.54 - 5.22) | 0.95 | 5.67 ± 1.28 (5.27 - 6.07) | 5.06 ± 1.09 (4.75 - 5.37) | 0.018 | 5.56 ± 1.28 (5.15 - 5.98) | 5.97 ± 1.05 (5.63 - 6.3) | 0.13 |
| **Salty** | 5.78 ± 1.56 (5.34 - 6.21) | 5.69 ± 1.65 (524 - 6.14) | 0.78 | 6.17 ± 1.37 (5.74 - 6.6) | 5.65 ± 1.56 (5.21 -6.1) | 0.1 | 5.89 ± 1.3 (5.47 - 6.31) | 6.05 ± 1.33 (5.62 - 6.47) | 0.6 |
| **Bitter** | 5.62 ± 1.5 (5.18 - 6.05) | 5.73 ± 1.72 (5.26. 6.2) | 0.73 | 6.22 ± 1.59 (5.92 - 6.72) | 5.59 ± 1.59 (5.13.6.05) | 0.02 | 5.83 ± 1.28 (3.42 - 6.25) | 6.31 ± 1.33 (5.89 - 6.74) | 0.11 |
| **TTS** | 22.94 ± 4.1 (21.8 - 24.07) | 23.01 ± 3.97 (21.93 - 24.1) | 0.92 | 25.27 ± 4.06 (24.01 - 26 53) | 22.95 ± 3.75 (21.88 - 24.03) | 0.007 | 24.64 ± 3.36 (23.56 - 25.73) | 25.94 ± 3.52 (24.82 - 27.06) | 0.1 |

| | ***Biochemical Assays*** | | | | | | | | |
|---|---|---|---|---|---|---|---|---|---|
| **Leptin (ng/ml)** | 26.89 (7.86) | 26.31 (14.74) | 0.28 | 18.19 (12.3) | 28.56 (17.63) | < 0.001 | 18.44 (12.78) | 21.38 (8.44) | 0.23 |
| **Ghrelin (pg/ml)** | 197.13 ± 42.13 (185.45 208.8) | 203.86 ± 37.74 (193.6 - 214.12) | 0.39 | 251.9 ± 52.31 (235.69-268.12) | 207.28 ± 37.82 (196.47 - 218.09) | < 0.001 | 232.51 ± 55.67 (214.57 - 250.45) | 250.83 ± 50.28 (234.84 - 266.82) | 0.13 |
| **IGF-1 (ng/ml)** | 159.51 ± 23.75 (152.92 -166.09) | 157.06 ± 26.38 (149.89 - 164.23) | 0.62 | 144.46 ± 27.01 (136.09 - 152.83) | 156.82 ± 24.87 (149.7 - 163.93) | 0.029 | 155.54 ± 32.12 (145.19 - 165.89) | 140.78 ± 23.7 (133.24 - 148.32) | 0.02 |
| **Serum glucose (mg/dl)** | 99 (13.75) | 99 (22.25) | 0.59 | 90 (12) | 98 (21.25) | 0.007 | 95 (18) | 87 (14.75) | 0.01 |
| **Insulin (µU/ml)** | 11.49 (7.51) | 10.61 (7.1) | 0.25 | 7.95 (5.4) | 11.41 (6.43) | < 0.001 | 9.6 (5.8) | 8.96 (6.01) | 0.48 |
| **Total cholesterol (mg/dl)** | 223.28 ± 38.36 (212.64 - 233.91) | 219.59 ± 36.3 (209.72 - 229.46) | 0.61 | 195.6 ± 31.19 (185.93 - 205.26) | 217.51 ± 33.44 (207.94 - 227.07) | 0.002 | 193.13 ± 34.23 (184.1 - 206.16) | 182.15 ± 30.09 (172.58 - 191.72) | 0.08 |
| **LDL (mg/dl)** | 142.32 ± 37.93(131.8 - 152.83) | 140.78 ± 35.27 (131.2 - 150.37) | 0.83 | 123.05 ± 24.48 (115.46 - 13063) | 138.93 ± 33.33 (129.4 - 148.46) | 0.014 | 127.64 ± 29.14 (118.25 - 137.03) | 100.21 ± 29.78 (90.74-109.68) | < 0.001 |
| **HDL (mg/dl)** | 55 (22.759 | 52 (19.25) | 0.4 | 57 (15.25) | 52(20) | 0.2 | 55 (11) | 60 (15) | 0.02 |
| **TGs (mg/dl)** | 111 (76.25) | 115 (41.25) | 0.69 | 91 (49.25) | 114 (36.75) | 0.07 | 94 (47) | 92.5 (55.5) | 0.63 |
| **Conjugated bilirubin (mg/dl)** | 0.16 ± 0.06 (0.14 - 0.18) | 0.18 ± 0.11 (0.15 - 0.21) | 0.22 | 0.17 ± 0.08 (0.14 - 0.2) | 0.2 ± 0.11 (0.17 - 0.23) | 0.18 | 0.17 ± 0.06 (0.14 - 0.19) | 0.17 ± 0.08 (0.14 - 0.2) | 0.81 |
| **Unconjugated bilirubin (mg/dl)** | 0.42 ± 0.19 (0.37 - 0.48) | 0.42 ± 0.22 (0.36 - 0.48) | 0.97 | 0.44 ± 0.2 (0.37- 0.5) | 0.45 ± 0.19 (0.4 - 0.51) | 0.74 | 0.41 ± 0.16 (0.36 - 0.46) | 0.42 ± 0.19 (0.36 - 0.49) | 0.7 |
| **ESR (mm/h)** | 12 (11.75) | 11 (10.25) | 0.73 | 7 (7.25) | 13 (12.25) | 0.017 | 10 (11) | 7 (9) | 0.12 |
| **CRP (mg/L)** | 0.9 (2.69) | 1.76 (2.62) | 0.79 | 0.5 (1.23) | 1.7 (1.46) | 0.005 | 0.7 (2.08) | 1.07 (1.47) | 0.48 |
| **AST (U/L)** | 23.32 ± 8.42 (20.98 - 25.65) | 23.25 ± 7.18 (21.29-25.2) | 0.96 | 19.25 ± 5.9 (17.41 - 21.08) | 23.53 ± 6.87 (21.61 - 25.53) | 0.002 | 19.32 ± 5.65 (17.5 - 21.14) | 18.68 ± 5.88 (16.81 - 20.55) | 0.63 |
| **ALT (U/L)** | 25.72 ± 11.85 (22.43 - 29) | 28.32 ± 13.56 (24.64 - 32.01) | 0.83 | 20.1 ± 9.91 (17.02 - 23.17) | 28.8 ± 14.09 (24.78 - 32.83) | 0.001 | 19.27 ± 9.33 (16.26 - 22.27) | 21.1 ± 10.13 (17.88 - 24.32) | 0.41 |
| **Uraemia (mg/dl)** | 35.36 ± 12.78 (31.81 - 38.9) | 35.17 ± 9.29 (32.64-37.69) | 0.93 | 32.33 = 9.89 (29.26 - 35.39) | 34.11 ± 7.95 (31.84 - 36.39) | 0.35 | 32.62 ± 9.22 (29.65 -35.59) | 27.86 ± 6.21 (25.89 - 29.84) | 0.01 |
| **Serum creatinine (mg/dl)** | 0.84 ± 0.15 (0.8 - 0.88) | 0.84 ± 0.15 (0.8 - 0.89) | 0.95 | 0.82 ± 0.15 (0.77 - 0.87) | 0.87 ± 0.16 (0.82 - 0.92) | 0.17 | 0.83 ± 0.15 (0.78 - 0.87) | 0.82 ± 0.15 (0.77 - 0.87) | 0.96 |
| **HOMA %B** | 102.97 ± 34.11(93.51 - 112.42) | 110.95 ± 45.34 (98.62 - 123.27) | 0.31 | 96.1 ± 34.03 (85.55 - 106.65) | 114.15 ± 44.53 (101.42 - 126.88) | 0.039 | 92.28 ± 30.13 (82.37 - 101.99 | 113.44 ± 44.23 (99.37 - 127.5 | 0.018 |
| **HOMA %S** | 76.68 ± 34.65 (67.07 - 86.28) | 76.15 ± 40.96 (65.01 - 87.28) | 0.94 | 113.88 ± 54.92 (96.86 - 130.9) | 69.78 ± 35.29 (59.69 - 79.87) | < 0.001 | 103.46 ± 54.02 (86.05 - 120.87 | 102.18 ± 59.49 (83.27 - 121.1 | 0.92 |
| **HOMA IR** | 1.56 ± 0.65 (1.38-1.74) | 1.71 ± 0.96 (1.45 - 1.97) | 0.36 | 1.07 ± 0.45 (0.92 - 1.21) | 1.8 ± 0.92 (1.54 - 2.06) | < 0.001 | 1.19 ± 0.48 (1.03 - 1.34 | 1.28 ± 0.63 (1.08 - 1.48) | 0.48 |

| | ***Anthropometric Variables*** | | | | | | | | |
|---|---|---|---|---|---|---|---|---|---|
| **WC (cm)** | 111.68 ± 10.38 (108.8 - 114.55) | 112.18 ± 9.6 (109.57 - 114.79) | 0.8 | 103.28 ± 9.77 (100.25 - 106.31) | 113.09 ± 8.98 (110.52 - 115.66) | < 0.001 | 105.07 ± 10.54 (101.68 - 108.47) | 107.59 ± 8.99 (104.73-110.45) | 0.26 |
| **Weight (kg)** | 94.57 ± 145 (9054. 98.59) | 97.07 ± 14.44 (93.15 - 101) | 0.38 | 86.16 ± 12.68 (82.23 - 90.09) | 97.07 ± 14.29 (92.98 - 101.16) | < 0.001 | 88.29 ± 13.62 (83.9-92.68) | 92.63 ± 14.68 (87.97 - 97.3) | 0.18 |
| **BMI (Kg/m²)** | 33.7 ± 4.38 (32.48 - 3491) | 33.87 ± 3.91 (32.8 - 34 93) | 0.83 | 30.89 ± 4.24 (29.57 - 32.2) | 33.88 ± 3.44 (32.9 - 34.87) | < 0.001 | 31.58 ± 4.74 (30.05 - 33.11) | 32.14 ± 3.43 (31.05 - 33.24) | 0.55 |
| **FM%*** | 39.72 ± 825 (37.43 - 42.01) | 40.95 ± 7.32 (38.96 - 42.94) | 0.42 | 28.1 ± 5.32 (26.44 - 29.75) | 40.87 ± 7.02 (38.86 - 42.88) | < 0.001 | 33.79 ± 8.15 (31.16-36.42) | 33.17 ± 5.76 (31.34-35.01) | 0.7 |
| **FM (Kg)*** | 37.41 ± 9.47 (34.78 - 40.04) | 39.7 ± 9.11 (37.22 - 42.18) | 0.21 | 24.05 ± 4.91 (22.53 - 25.57) | 39.53 ± 8.32 (37.15 - 41.91) | < 0.001 | 29.79 ± 8.36 (27.1 - 32.49) | 30.65 ± 6.76 (28.5 - 32.8) | 0.02 |
| **MM%*** | 26.51 ± 4.33 (25.31 - 27.71) | 25.9 ± 4.24 (24.75 - 27.05) | 0.47 | 36.62 ± 7.67 (34.24 - 39) | 26.24 ± 4.32 (25 - 27.47) | < 0.001 | 31.1 ± 5.05 (29.48 - 32.73) | 33.2 ± 4.71(31.7-34.7) | 0.06 |
| **MM (Kg)*** | 25.16 ± 5.9 (23.53 - 26.8) | 25.22 ± 5.86 (23.62 - 26.81) | 0.96 | 31.7 ± 8.61 (29.03 - 34.37) | 25.56 ± 6.07 (23.82 - 27.29) | < 0.001 | 27.58 ± 6.57 (25.46 - 29.7) | 30.72 ± 6.07 (28.79 - 32.65) | 0.03 |
| **VFLevel*** | 15.38 ± 4.93 (14.01 - 16.74) | 14.4 ± 4.68 (13.13 - 15.67) | 0.3 | 12.22 ± 4.13 (10.94 - 13.5) | 14.4 ± 4.64 (13.07 - 15.73) | 0.02 | 13 ± 4.51 (11.54 - 14.45) | 11.94 ± 4.21 (10.6 - 13.28) | 0.3 |

### Changes in cardiometabolic and anthropometric variables

### Changes from baseline

After the 6-month FMD period, significant decreases were found for FMD4Control participants (n = 40) in leptin, IGF-1, total cholesterol, low-density lipoprotein (LDL), serum glucose, insulin, insulin sensitivity (HOMA %S), Homeostasis Model Assessment of insulin resistance (HOMA IR), aspartate aminotransferase (AST), alanine aminotransferase (ALT), waist circumference (WC), estimated fat mass (FM) and visceral fat (VF) level with a significant increase in ghrelin and estimated muscle mass (MM) (Omron HBF-500 BIA, Omron Medizintechnik, Mannheim, Germany) compared to baseline values at T0 (Table 3, Figure 3). Similar changes were also found when comparing Control->FMD participants (n = 38) at T2 with partecipants at T1, whereas - as expected - no significant differences were found in Control→FMD group (n = 47) when comparing said partecipants at T1 and at T0 (control period) (Table 3, Figure 3). The comparison between changes in all the participants after FMD treatment (FMD4Control + Control->FMD) and Control→FMD participants during the first 6-month period (routinary eating) highlighted a significant decrease in leptin, IGF-1, total cholesterol, LDL, triglycerides (TGs), erythrocyte sedimentation rate (ESR), C-reactive protein (CRP), serum glucose, insulin, HOMA IR, AST, ALT, uraemia and serum creatinine in patients after the 6 monthly FMD cycles, whereas a significant increase was found in ghrelin and HOMA %S (Table 3, Figure 2). Finally, a significant decrease in WC, weight, BMI, FM (% and KG) and VF level and a significant increase in MM were found in the same comparison (Table 3).

The following variables were found to be significantly different when also comparing values at T2 with values at T0 in the FMD4Control participants (n = 37): leptin (p = 0.006), ghrelin (p = 0.003), total cholesterol (p < 0.001), insulin (p = 0.002), ALT (p = 0.005), HOMA %S (p = 0.008), HOMA IR (p = 0.002), estimated FM (p = 0.006 and p = 0.001 for % and Kg, respectively) and estimated MM% (p < 0.001).

No significant differences were found in IGF-1 (p = 0.24), high density lipoprotein (HDL) (p = 0.65), LDL (p = 0.012), TGs (p = 0.06), AST (p = 0.015), ESR (p = 0.3), CRP (p = 0.19), serum glucose (p = 0.13), conjugated (p = 0.49) and unconjugated (p = 0.86) bilirubin, uraemia (p = 0.21), serum creatinine (p = 0.23), steady state beta cell function (HOMA %B) (p = 0.14), weight (p = 0.12), WC (p = 0.013), BMI (p = 0.1), estimated MM Kg (p = 0.05) and VF level (p = 0.02) in the same comparison. After the second 6-months period in which FMD4Control participants returned to their dietary habits (T2), compared to T1 values no significant differences were found in leptin (p = 0.23), IGF-1 (p = 0.16), ghrelin (p = 0.13), serum glucose (p = 0.19), insulin (p = 0.27), total cholesterol (p = 0.98), LDL (p = 0.52), HDL (p = 0.63), TGs (p = 0.75), ESR (p = 0.3), CRP (p = 0.42), conjugated (p = 0.83) and unconjugated bilirubin (p = 0.47), AST (p = 0.93), ALT (p = 0.83), uraemia (p = 0.85) and serum creatinine (p = 0.92), HOMA %B (p = 0.74), HOMA %S (p = 0.39), HOMA IR (p = 0.23), WC (p = 0.54), weight (p = 0.51), BMI (p = 0.49) and VF level (p = 0.59). A significant increase and decrease respectively in estimated FM (p < 0.001 and p < 0.001 for % and Kg, respectively) and MM (p < 0.001 and p = 0.013 for % and Kg, respectively) was found.

As shown for the FMD4Control group, in the Control→FMD group (n = 38), when comparing the values at T2 with the values at T1, a significant decrease of leptin (p < 0.001), IGF-1 (p = 0.004), serum glucose (p < 0.001), insulin (p = 0.002), total cholesterol (p < 0.001), LDL (p < 0.001), AST (p = 0.002), ALT (p = 0.009) and uraemia (p < 0.001), HOMA IR (p = 0.002) as well as in WC (p < 0.001), BMI (p = 0.006) and estimated FM (p < 0.001 for both % and Kg) and VF levels (p = 0.002) resulted. Furthermore, a significant increase was shown for ghrelin (p < 0.001), HDL (p = 0.002), HOMA %S (p = 0.005) and estimated MM (p = 0.001 and p = 0.002 for % and Kg, respectively). No significant differences were found in TGs (p = 0.03), ESR (p = 0.018), CRP (p = 0.3), conjugated (p = 0.09) and unconjugated bilirubin (p = 0.36), serum creatinine (p = 0.16), HOMA %B (p = 0.79) and weight (p = 0.06) (Table 3, Figure 3). Many of these parameters were found to be significantly decreased when also comparing the values at T2 with the values at T0: leptin (p < 0.001), serum glucose (p = 0.001), insulin (p = 0.002), total cholesterol (p < 0.001), LDL (p < 0.001), AST (p = 0.002), ALT (p = 0.011) and uraemia (p < 0.001), HOMA IR (p = 0.003) as well as WC (p = 0.002), BMI (p = 0.006) and estimated FM (p < 0.001 for both % and Kg) and VF levels (p = 0.001); on the contrary, a significant increase in ghrelin (p < 0.001), HOMA %S (p = 0.008) and estimated MM (p < 0.001 for both % and Kg) was found. No significant differences were found in TGs (p = 0.19), ESR (p = 0.02), CRP (p = 0.39), conjugated (p = 0.28) and unconjugated bilirubin (p = 0.78), serum creatinine (p = 0.27), HOMA %B (p = 0.67), weight (p = 0.06), IGF-1 (p = 0.019) and HDL (p = 0.0167).

### Between-group analysis

The between-group analysis showed that after six FMD cycles FMD4Control participants exhibited a significant reduction in serum leptin (p < 0.001), insulin (p < 0.001), glucose (p = 0.007), total cholesterol (p = 0.002), LDL (p = 0.014), CRP (p = 0.005), AST (p = 0.002) and ALT (p = 0.001), HOMA IR (p < 0.001) levels and an increase in ghrelin serum (p < 0.001) and HOMA %S (p < 0.001) levels with respect to Control→FMD on the control diet. A trend for a reduced level was observed for IGF-1 (p = 0.029), and ESR (p = 0.017), but no significant differences were found for HDL (p = 0.2), TGs (p = 0.07), conjugated (p = 0.18) and unconjugated (p = 0.74) bilirubin, uraemia (p = 0.35), serum creatinine (p = 0.17) and HOMA %B (p = 0.039).

FMD4Control participants displayed a significantly lower WC (p < 0.001), weight (p < 0.001), BMI (p < 0.001) and estimated FM (p < 0.001 for both % and Kg) and a higher estimated MM (p < 0.001 for both % and Kg) but only a trend for a lower VF level (p = 0.02) (Table 4).

After the second 6-months period during which Control→FMD participants underwent the FMD intervention and FMD4Control participants followed their dietary habits and no FMD, Control→FMD participants LDL, serum glucose and uraemia were found to be significantly lower with respect to FMD4Control (p < 0.001, p = 0.01 and p = 0.01, respectively). A trend for a reduced level was observed for IGF-1 (p = 0.02), but no significant differences were observed for HDL (p = 0.02), leptin (p = 0.23), ghrelin (p = 0.13), insulin (p = 0.48), total cholesterol (p = 0.08) TGs (p = 0.63), ESR (p = 0.12), CPR (p = 0.48), conjugated (p = 0.81) and unconjugated (p = 0.7) bilirubin, AST (p = 0.63), ALT (p = 0.41) and serum creatinine (p = 0.96), HOMA %B (p = 0.018), HOMA %S (p = 0.92), HOMA IR (p = 0.48) nor in WC (p = 0.26), weight (p = 0.18), BMI (p = 0.55) as well as in estimated FM (p = 0.7 and p = 0.62 for % and Kg, respectively), MM (p = 0.06 and p = 0.03 for % and Kg, respectively) and VF levels (p = 0.3) (Table 4).

### Discussion

The present disclosure shows significant - parallel - improvement in taste and smell sensitivity after FMD cycles in both between-group and within-subject analysis (Table 3, Table 4 Figure 2, Figure 3, Figure 4). OT, OI, TDI, sweet and TTS were the main outcome variables found to be significantly improved in FMD4Control after the 6-month FMD period when compared to the control diet group. Similar results were observed in Control->FMD after cross over and 6 cycles of FMD (Table 3). Notably, the portion of patients with hyposmia was reduced by nearly 6 fold with long-lasting effects of FMD cycles on taste and smell sensitivity (Table 3, Figure 2).

Notably, the many improvements maintained after 6 months from the end of the FMD cycles raise the possibility that FMD cycles, periodically followed during a year, and possibly only every 3-4 months, could be at least partially effective while rendering this approach even more feasible by requiring a change in dietary habits for only 15-20 days per year.

Additionally, ghrelin, leptin and insulin continued to be significantly different from baseline in FMD4Control after the follow up 6-months period.

Further, these results provide strong evidence for the ability of FMD cycles to improve taste and smell function, while also reducing many risk factors for cardiometabolic diseases not only causing by weight loss.

FMD cycles also reduced metabolic and inflammatory markers and reduced drug use in diabetic patients, wherein most of the chemosensory and metabolic changes are not correlated with weight loss. In summary, this disclosure provides evidence for the effect of periodic cycles of FMD in improving chemosensory function, while reducing cardiometabolic risk factors, without requiring long-term lifestyle changes.

### Biobliography

1. Berthoud, H.R., and Zheng, H. (2012). Modulation of taste responsiveness and food preference by obesity and weight loss. Physiol Behav 107, 527-532. 10.1016/j.physbeh.2012.04.004.
2. Peng, M., Coutts, D., Wang, T., and Cakmak, Y.O. (2019). Systematic review of olfactory shifts related to obesity. Obes Rev 20, 325-338. 10.1111/obr. 12800.
3. Micarelli, A., Malacrida, S., Strapazzon, G., Mrakic-Sposta, S., Micarelli, B., Alessandrini, N., Carbini, V., Caputo, S., Falla, M., and Alessandrini, M. (2021). Impact of Nutritional Intervention on Taste Perception-A Scoping Review. Foods 10. 10.3390/foods10112747.
4. Poessel, M., Breuer, N., Joshi, A., Pampel, A., Villringer, A., Hummel, T., and Horstmann, A. (2020). Reduced Olfactory Bulb Volume in Obesity and Its Relation to Metabolic Health Status. Front Hum Neurosci 14, 586998. 10.3389/fnhum.2020.586998.
5. Micarelli, A., Vezzoli, A., Malacrida, S., Micarelli, B., Misici, I., Carbini, V., Iennaco, I., Caputo, S., Mrakic-Sposta, S., and Alessandrini, M. (2023). Taste Function in Adult Humans from Lean Condition to Stage II Obesity: Interactions with Biochemical Regulators, Dietary Habits, and Clinical Aspects. Nutrients 15. 10.3390/nu15051114.
6. Makaronidis, J.M., Neilson, S., Cheung, W.H., Tymoszuk, U., Pucci, A., Finer, N., Doyle, J., Hashemi, M., Elkalaawy, M., Adamo, M., et al. (2016). Reported appetite, taste and smell changes following Roux-en-Y gastric bypass and sleeve gastrectomy: Effect of gender, type 2 diabetes and relationship to post-operative weight loss. Appetite 107, 93-105. 10.1016/j.appet.2016.07.029.
7. Micarelli, A., Mrakic-Sposta, S., Micarelli, B., Malacrida, S., Misici, I., Carbini, V., Iennaco, I., Caputo, S., Vezzoli, A., and Alessandrini, M. (2022). Smell Impairment in Stage I-II Obesity: Correlation with Biochemical Regulators and Clinical Aspects. Laryngoscope 132, 2028-2035. 10.1002/lary.30325.
8. Velluzzi, F., Deledda, A., Onida, M., Loviselli, A., Crnjar, R., and Sollai, G. (2022). Relationship between Olfactory Function and BMI in Normal Weight Healthy Subjects and Patients with Overweight or Obesity. Nutrients 14. 10.3390/nu14061262.
9. Espinoza Garcia AS, Martinez Moreno AG, Reyes Castillo Z. The role of ghrelin and leptin in feeding behavior: genetic and molecular evidence. Endocrinologia, Diabetes y Nutrición (English ed). 2021;68(9):654-663. doi:10.1016/j.endien.2020.10.009
10. Longo, V.D., and Panda, S. (2016). Fasting, Circadian Rhythms, and Time-Restricted Feeding in Healthy Lifespan. Cell Metab 23, 1048-1059. 10.1016/j.cmet.2016.06.001.
11. Brandhorst, S. (2021). Fasting and fasting-mimicking diets for chemotherapy augmentation. Geroscience 43, 1201-1216. 10.1007/s11357-020-00317-7.
12. Wei, M., Brandhorst, S., Shelehchi, M., Mirzaei, H., Cheng, C.W., Budniak, J., Groshen, S., Mack, W.J., Guen, E., Di Biase, S., et al. (2017). Fasting-mimicking diet and markers/risk factors for aging, diabetes, cancer, and cardiovascular disease. Sci Transl Med 9. 10.1126/scitranslmed.aai8700.
13. Sulaj, A., Kopf, S., von Rauchhaupt, E., Kliemank, E., Brune, M., Kender, Z., Bartl, H., Cortizo, F.G., Klepac, K., Han, Z., et al. (2022). Six-Month Periodic Fasting in Patients With Type 2 Diabetes and Diabetic Nephropathy: A Proof-of-Concept Study. J Clin Endocrinol Metab 107, 2167-2181. 10.1210/clinem/dgac197.
14. Vignini, A., Borroni, F., Sabbatinelli, J., Pugnaloni, S., Alia, S., Taus, M., Ferrante, L., Mazzanti, L., and Fabri, M. (2019). General Decrease of Taste Sensitivity Is Related to Increase of BMI: A Simple Method to Monitor Eating Behavior. Dis Markers 2019, 2978026. 10.1155/2019/2978026.
15. Landis, B.N., Welge-Luessen, A., Brämerson, A., Bende, M., Mueller, C.A., Nordin, S., and Hummel, T. (2009). "Taste Strips" - a rapid, lateralized, gustatory bedside identification test based on impregnated filter papers. J Neurol 256, 242-248. 10.1007/s00415-009-0088-y.
16. Maloh, J., Wei, M., Hsu, W.C., Caputo, S., Afzal, N., and Sivamani, R.K. (2023). The Effects of a Fasting Mimicking Diet on Skin Hydration, Skin Texture, and Skin Assessment: A Randomized Controlled Trial. J Clin Med 12. 10.3390/jcm12051710.
17. Hummel, T., Kobal, G., Gudziol, H., and Mackay-Sim, A. (2007). Normative data for the "Sniffin' Sticks" including tests of odor identification, odor discrimination, and olfactory thresholds: an upgrade based on a group of more than 3,000 subjects. Eur Arch Otorhinolaryngol 264, 237-243. 10.1007/s00405-006-0173-0.
18. Hummel, T., Sekinger, B., Wolf, S.R., Pauli, E., and Kobal, G. (1997). 'Sniffin' sticks': olfactory performance assessed by the combined testing of odor identification, odor discrimination and olfactory threshold. Chem Senses 22, 39-52. 10.1093/chemse/22.1.39.
19. Hedner, M., Larsson, M., Arnold, N., Zucco, G.M., and Hummel, T. (2010). Cognitive factors in odor detection, odor discrimination, and odor identification tasks. J Clin Exp Neuropsychol 32, 1062-1067. 10.1080/13803391003683070.
20. Rumeau, C., Nguyen, D.T., and Jankowski, R. (2016). How to assess olfactory performance with the Sniffin' Sticks test(®). Eur Ann Otorhinolaryngol Head Neck Dis 133, 203-206. 10.1016/j.anorl.2015.08.004.
21. Campolo, J., Corradi, E., Rizzardi, A., Parolini, M., Dellanoce, C., Di Guglielmo, M.L., Tarlarini, P., Cattaneo, M., Trivella, M.G., and De Maria, R. (2021). Correlates of olfactory impairment in middle-aged non-diabetic Caucasian subjects with stage I-II obesity. Eur Arch Otorhinolaryngol 278, 2047-2054. 10.1007/s00405-020-06442-5.
22. Oleszkiewicz, A., Schriever, V.A., Croy, I., Hähner, A., and Hummel, T. (2019). Updated Sniffin' Sticks normative data based on an extended sample of 9139 subjects. Eur Arch Otorhinolaryngol 276, 719-728. 10. 1007 j s00405-0 18-5248-1.
23. Mueller, C., Kallert, S., Renner, B., Stiassny, K., Temmel, A.F., Hummel, T., and Kobal, G. (2003). Quantitative assessment of gustatory function in a clinical context using impregnated "taste strips". Rhinology 41, 2-6.
24. Matthews, D.R., Hosker, J.P., Rudenski, A.S., Naylor, B.A., Treacher, D.F., and Turner, R.C. (1985). Homeostasis model assessment: insulin resistance and beta-cell function from fasting plasma glucose and insulin concentrations in man. Diabetologia 28, 412-419. 10.1007/bf00280883.
25. Poessel, M., Morys, F., Breuer, N., Villringer, A., Hummel, T., and Horstmann, A. (2022). Brain response to food odors is not associated with body mass index and obesity-related metabolic health measures. Appetite 168, 105774. 10.1016/j.appet.2021.105774.
26. Aandstad, A., Holtberget, K., Hageberg, R., Holme, I., and Anderssen, S.A. (2014). Validity and reliability of bioelectrical impedance analysis and skinfold thickness in predicting body fat in military personnel. Mil Med 179, 208-217. 10.7205/milmed-d-12-00545.
27. Betts, J.A., Smith, H.A., Johnson-Bonson, D.A., Ellis, T.I., Dagnall, J., Hengist, A., Carroll, H., Thompson, D., Gonzalez, J.T., and Afman, G.H. (2019). The Energy Cost of Sitting versus Standing Naturally in Man. Med Sci Sports Exerc 51, 726-733. 10.1249/mss.0000000000001841.
28. Pasanta, D., Htun, K.T., Pan, J., Tungjai, M., Kaewjaeng, S., Chancharunee, S., Tima, S., Kim, H.J., Kaewkhao, J., and Kothan, S. (2021). Waist Circumference and BMI Are Strongly Correlated with MRI-Derived Fat Compartments in Young Adults. Life (Basel) 11. 10.3390/life11070643.
29. Wise, P.M., Nattress, L., Flammer, L.J., and Beauchamp, G.K. (2016). Reduced dietary intake of simple sugars alters perceived sweet taste intensity but not perceived pleasantness. Am J Clin Nutr 103, 50-60. 10.3945/ajcn.115.112300.
30. Cattaneo, C., Mambrini, S.P., Gilardini, L., Scacchi, M., Pagliarini, E., and Bertoli, S. (2023). Impact of 4-week of a restricted Mediterranean diet on taste perception, anthropometric, and blood parameters in subjects with severe obesity. Front Nutr 10, 1196157. 10.3389/fnut.2023.1196157.

## Claims

1. A diet composition for use in the prevention and/or the treatment of hyposmia and/or hypogeusia in a human subject, the diet composition comprising:
a fasting mimicking diet component to be administered for a first time period, said fasting mimicking diet component providing less than 50% of the normal caloric intake of the subject with both protein restriction and sugar restriction; and
a re-feeding diet component to be administered for a second time period, said re-feeding diet component providing 60-100% of the normal caloric intake of the subject;
wherein the fasting mimicking diet component and the re-feeding diet component are administered for multiple cycles.

2. The diet composition for the use according to claim 1, wherein the diet composition is administered to a human subject with Body Mass Index (BMI) ≥ 25, preferably a BMI equal to or higher than 25 and lower than 40.

3. The diet composition for the use according to claim 1 or 2, wherein the first time period is from 2 days to 10 days, preferably 2 to 6 days, more preferably the first time period being 5 days, and/or said second time period is from 7 to 85 days, preferably 25-26 days.

4. The diet composition for the use according to any one of claims 1-3, wherein said fasting mimicking diet component and said re-feeding diet component are administered for multiple cycles for at least 6 months, preferably for 6-12 months.

5. The diet composition for the use according to any one of claims 1-4, wherein the blood concentration of leptin is reduced by 5 to 18 ng/ml and/or the blood concentration of ghrelin is increased by 30 to 90 pg/ml, as measured after 6 months from the start of the administration of said diet composition for multiple cycles, when compared to the blood concentration of leptin and/or ghrelin as measured before starting the administration of said diet composition, respectively.

6. The diet composition for the use according to any one of claims 1-5, wherein said fasting mimicking diet component provides the subject with no more than 1200 kcal/day, preferably providing to the subject 600-1100 kcal/day.

7. The diet composition for the use according to claim 6, wherein the first time period is 5 days and said fasting mimicking diet component provides the subject with 800-1200 kcal/day for the first day of said first time period and with 600-800 kcal/day for the second to the fifth day of said first time period.

8. The diet composition for the use according to any one of claims 1-7, wherein said fasting mimicking diet component provides the subject with a protein amount less than or equal to 36 g/day, preferably 0-20 g/day.

9. The diet composition for the use according to any one of claims 1-8, wherein said fasting mimicking diet component provides the subject with no more than 11 kcal/kg of body weight/day, preferably 2-8 kcal/kg of body weight/day.

10. The diet composition for the use according to any one of claims 1-9, wherein said fasting mimicking diet component comprises proteins in an amount that is less than 15%, preferably less than 12%, of the total calories provided by the fasting mimicking diet component.

11. The diet composition for the use according to any one of claims 1-10, wherein said fasting mimicking diet component comprises sugars in an amount that is less than 50%, preferably less than 48%, of the total calories provided by the fasting mimicking diet component.

12. The diet composition for the use according to any one of claims 1-11, wherein said fasting mimicking diet component comprises fats in an amount that is equal to or more than 40%, preferably less than 50%, more preferably less than 48%, of the total calories provided by the fasting mimicking diet component.

13. The diet composition for the use according to any one of claims 1-12, wherein said fasting mimicking diet component comprises at least 45% calories from fatty acids and up to 5% calories from plant-based proteins and a maximum of 50% calories from carbohydrates.

14. A diet composition for use in the prevention and/or the treatment of a disease or disorder or condition involving a leptine and/or ghrelin imbalance in a human subject, the diet composition comprising:
a fasting mimicking diet component to be administered for a first time period, said fasting mimicking diet component providing less than 50% of the normal caloric intake of the subject with both protein restriction and sugar restriction; and
a re-feeding diet component to be administered for a second time period, said re-feeding diet component providing 60-100% of the normal caloric intake of the subject;
wherein the fasting mimicking diet component and the re-feeding diet component are administered for multiple cycles.

15. The diet composition for the use according to claim 14, wherein said disease or disorder or condition is selected from overweight or obesity.
